# EUROPEAN PATENT APPLICATION

(11) **EP 4 434 960 A1**
(43) Date of publication of application: **25.09.2024**
(21) Application number: 22895579.5
(22) Date of filing: 14.11.2022
(51) Int. Cl.: C07B 59/00, B01J 27/053, B01J 29/08, B01J 29/18, B01J 29/40, B01J 29/60, B01J 29/65, B01J 29/70, B01J 31/04, C07B 61/00, C07C 37/50, C07C 39/04, C07C 39/08, C07C 209/68, C07C 211/46

(54) **CYCLIC COMPOUND AND METHOD FOR PRODUCING CYCLIC COMPOUND**

(30) Priority: 16.11.2021 JP 2021186562; 16.11.2021 JP 2021186563; 16.11.2021 JP 2021186564
(71) Applicant: Sumitomo Bakelite Co., Ltd., Shinagawa-ku Tokyo 140-0002 (JP)
(72) Inventor: INOUE, Yusuke, Tokyo 140-0002 (JP); MURATA, Ryuichi, Tokyo 140-0002 (JP); HASHIZUME, Masayoshi, Tokyo 140-0002 (JP); FUJIWARA, Daisuke, Tokyo 140-0002 (JP); TACHIBANA, Kenya, Tokyo 140-0002 (JP)
(74) Representative: Ter Meer Steinmeister & Partner
(86) International application number: PCT/JP2022/042272
(87) International publication number: WO 2023/090298

(57) **Abstract**

An embodiment of a method for producing a cyclic compound of the present invention includes a step of causing a decarboxylation reaction in a cyclic carboxylic acid compound by subjecting a starting material containing the cyclic carboxylic acid compound to a heat treatment of heating under a catalyst to obtain a cyclic compound, in which the catalyst includes zeolite formed of an aluminum/silicon-containing composite oxide, and in which the zeolite has an SiO₂/Al₂O₃ ratio of 7 to 500. In addition, another embodiment of the method for producing a cyclic compound of the present invention includes a step of causing a decarboxylation reaction in a cyclic carboxylic acid compound by subjecting a starting material containing the cyclic carboxylic acid compound to a heat treatment of heating in the presence of a non-solid acid to obtain a cyclic compound.

## Description

### Technical Field

The present invention relates to a cyclic compound and a method for producing a cyclic compound.

### Background Art

A cyclic compound having a specific structure is used, for example, as a starting material for various applications such as chemical products, fragrances, cosmetics, pharmaceuticals, and agricultural chemicals.

PTL 1 discloses a method for synthesizing a hydroxybenzene compound by decarboxylating a hydroxybenzoic acid under a catalyst as a method for producing a hydroxybenzene compound. In addition, PTL 1 discloses, as a catalyst, a zeolite made of an aluminum/silicon-containing composite oxide and having an FAU structure.

However, the production method disclosed in PTL 1 does not provide a sufficient yield of hydroxybenzene compounds. Therefore, there is a need to improve the yield. In addition, even if the yield is high, in a case where the quality of the produced hydroxybenzene compound is low, for example, in a case where a degree of coloring is large, the hydroxybenzene compound cannot be used effectively.

PTL 2 discloses a method for producing an aromatic compound from an aromatic carbon resource through an oxidation step and a decarboxylation step. In addition, PTL 1 discloses that a decarboxylation step is carried out by using a cuprous oxide as a catalyst and carrying out a heat treatment in subcritical water in the presence of the catalyst. The cuprous oxide is a solid and can be easily recovered, and thus can be reused. Therefore, the method disclosed in PTL 2 can reduce costs compared to, for example, a method using a noble metal as a catalyst.

In addition, in the method disclosed in PTL 2, reaction efficiency of the decarboxylation reaction is increased by carrying out the heat treatment under high pressure.

However, the catalyst disclosed in PTL 2 is considered to be a solid even under heating. Therefore, the method disclosed in PTL 2 has an issue in that it is difficult to increase contact efficiency between the aromatic carbon resource and the catalyst. In addition, using subcritical water is expected to increase the reaction efficiency of the decarboxylation reaction, but maintaining subcritical water requires equipment that can maintain a high pressure, and thus high equipment costs are inevitable.

PTL 3 discloses a phenolic resin having a structure derived from plant-derived phenols. By providing a structure derived from plant-derived phenols, a phenolic resin with a high plant-derived rate can be obtained. In addition, PTL 3 discloses that by preparing a rubber composition containing such a phenolic resin and a rubber component, a rubber composition with improved rubber strength can be obtained.

In addition, PTL 3 discloses that a proportion of structures derived from plant-derived phenols in the phenolic resin is 50% to 65% by mass. By including the structure derived from plant-derived phenols in such a proportion, it is possible to obtain a phenolic resin with a high plant-derived rate of the phenolic resin and improved compatibility with rubber.

However, in the phenolic resin disclosed in PTL 3, the proportion of structures derived from plant-derived phenols is 50% to 65% by mass. In a case where the plant-derived rate of the phenolic resin is at this level, an effect of suppressing an increase in carbon dioxide in the atmosphere at a time of disposal, that is, the contribution to carbon neutrality, is limited.

In addition, plant-derived phenols often contain miscellaneous components. Therefore, plant-derived phenols have an issue that the quality tends to be unstable.

### Citation List

### Patent Literature

[PTL 1]: JP2016-23136A
[PTL 2]: JP2016-179950A
[PTL 3]: JP2017-119765A

### Summary of Invention

### Technical Problem

An object of the present invention is to provide a method for producing a cyclic compound that can produce a high-quality cyclic compound in a high yield from a cyclic carboxylic acid compound.

In addition, another object of the present invention is to provide a cyclic compound that greatly contributes to carbon neutrality and has stable quality, and a method for producing the same.

### Solution to Problem

Such objects can be achieved by the present invention described in the following items (1) to (20).
(1) A method for producing a cyclic compound, including: a step of causing a decarboxylation reaction in a cyclic carboxylic acid compound by subjecting a starting material containing the cyclic carboxylic acid compound to a heat treatment of heating under a catalyst to obtain a cyclic compound,
   in which the catalyst includes zeolite formed of an aluminum/silicon-containing composite oxide, and
   in which the zeolite has an SiO₂/Al₂O₃ ratio of 7 to 500.
(2) The method for producing a cyclic compound according to (1), in which an acid amount of the zeolite determined by an NH₃-TPD method is 0.1 to 2.5 [mmol/g].
(3) The method for producing a cyclic compound according to (1) or (2), in which the catalyst includes at least one of FER type zeolite, MFI type zeolite, BEA type zeolite, and MOR type zeolite.
(4) The method for producing a cyclic compound according to any one of (1) to (3), in which the zeolite has a pore diameter of 0.4 nm or more and 1.5 nm or less.
(5) The method for producing a cyclic compound according to any one of (1) to (4), in which the zeolite contains hydrogen.
(6) A method for producing a cyclic compound, including: a step of causing a decarboxylation reaction in a cyclic carboxylic acid compound by subjecting a starting material containing the cyclic carboxylic acid compound to a heat treatment of heating in a presence of a non-solid acid to obtain a cyclic compound,
   in which the non-solid acid has an acid dissociation constant pKa of 5.00 or less.
(7) The method for producing a cyclic compound according to (6), in which the non-solid acid includes an inorganic acid.
(8) The method for producing a cyclic compound according to (6) or (7), in which the non-solid acid includes an organic acid.
(9) The method for producing a cyclic compound according to (8), in which the organic acid is an aliphatic carboxylic acid or a derivative of the aliphatic carboxylic acid.
(10) The method for producing a cyclic compound according to (6), in which the heat treatment is further carried out in a presence of a solid acid.
(11) The method for producing a cyclic compound according to any one of (6) to (10), in which a temperature of the heat treatment is 150°C to 350°C.
(12) The method for producing a cyclic compound according to any one of (1) to (11), in which the starting material does not include a solvent.
(13) The method for producing a cyclic compound according to any one of (1) to (12), in which the cyclic carboxylic acid compound is 4-hydroxybenzoic acid, 3,4-dihydroxybenzoic acid, 4-aminobenzoic acid, or shikimic acid.
(14) The method for producing a cyclic compound according to any one of (1) to (13), in which the starting material is derived from biomass.
(15) A cyclic compound produced by causing a decarboxylation reaction in a cyclic carboxylic acid compound derived from biomass by subjecting a starting material containing the cyclic carboxylic acid compound to a heat treatment,
   in which a concentration of radioactive carbon ¹⁴C in all carbon atoms is 80% to 100%, when a concentration of radioactive carbon ¹⁴C in circulating carbon as of 1950 is set to 100%, and
   in which a proportion of a main component having a single molecular structure is 90 to 100 mol%.
(16) The cyclic compound according to (15), in which the main component is an aromatic compound derived from biomass.
(17) The cyclic compound according to (15) or (16), in which a temperature of the heat treatment is 150°C to 350°C.
(18) The cyclic compound according to any one of (15) to (17), in which the starting material does not include a solvent.
(19) The cyclic compound according to any one of (15) to (18), in which the cyclic carboxylic acid compound is 4-hydroxybenzoic acid, 3,4-dihydroxybenzoic acid, 4-aminobenzoic acid, or shikimic acid.
(20) A method for producing a cyclic compound, including: a step of causing a decarboxylation reaction in a cyclic carboxylic acid compound derived from biomass by subjecting a starting material containing the cyclic carboxylic acid compound to a heat treatment to obtain a cyclic compound,
   in which a concentration of radioactive carbon ¹⁴C in all carbon atoms of the cyclic compound is 80% to 100%, when a concentration of radioactive carbon ¹⁴C in circulating carbon as of 1950 is 100%, and
   in the cyclic compound, a proportion of a main component having a single molecular structure is 90 to 100 mol%.

Advantageous Effects of Invention

According to the present invention, a high-quality cyclic compound can be produced from a cyclic carboxylic acid compound in a high yield.

In addition, according to another embodiment of the present invention, a cyclic compound that greatly contributes to carbon neutrality and has stable quality can be obtained.

In addition, according to another embodiment of the present invention, the cyclic compound can be produced.

### Brief Description of Drawings

FIG. 1 is a step diagram for explaining a method for producing a cyclic compound according to each embodiment of the present invention.
FIG. 2 is a distribution diagram created by plotting a yield and a degree of coloring of a cyclic compound produced in each Example and each Comparative Example on an orthogonal coordinate system.

### Description of Embodiments

Hereinafter, the method for producing a cyclic compound of the present invention will be described based on suitable embodiments shown in the accompanying drawings.

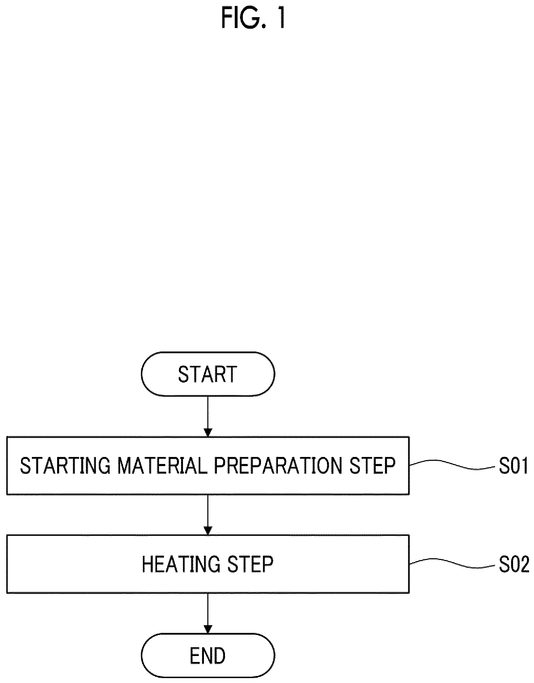

FIG. 1 is a step diagram for explaining a method for producing a cyclic compound according to each embodiment of the present invention.

### <First embodiment>

First, a first embodiment of the method for producing a cyclic compound of the present invention will be described.

The method for producing a cyclic compound according to the present embodiment includes at least a step of causing a decarboxylation reaction in a cyclic carboxylic acid compound by subjecting a starting material containing the cyclic carboxylic acid compound to a heat treatment of heating under a catalyst to obtain a cyclic compound. Here, as an example, an embodiment including a starting material preparation step S01, which is an optional step, and a heating step S02 described above will be described.

### 1. Starting material preparation step S01

In the starting material preparation step S01, a starting material containing a cyclic carboxylic acid compound is prepared. The present step may be carried out depending on the necessity, and can be omitted in a case where a product has already been prepared.

The cyclic carboxylic acid compound is a general term for a compound having a cyclic structure and a carboxy group bonded to the cyclic structure. The cyclic carboxylic acid compound is, for example, a compound represented by Formula (1).

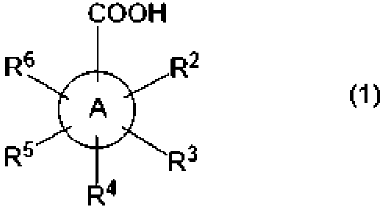

[In the formula, ring A is a 5-membered ring of a saturated ring, a partially saturated ring, or an aromatic ring, a 6-membered ring of a saturated ring, a partially saturated ring, or an aromatic ring, or a fused ring containing a 5-membered ring or a 6-membered ring. R² to R⁶ (R² to R⁵ in a case where ring A is 5-membered ring) are each independently a hydrogen atom, a hydroxyl group, an amino group, an alkoxy group, a carboxy group, or a carbonyl group.]

Examples of the 5-membered ring of a saturated ring, a partially saturated ring, or an aromatic ring include a furan structure, a thiophene structure, a pyrrole structure, a pyrrolidine structure, a tetrahydrofuran structure, a 2,3-dihydrofuran structure, a pyrazole structure, an imidazole structure, an oxazole structure, an isoxazole structure, a thiazole structure, an isothiazole structure, and the like.

Examples of the 6-membered ring of a saturated ring include a hydrocarbon-based saturated ring such as a cyclohexane structure, a nitrogen-containing saturated ring such as a piperidine structure, a piperazine structure, a triazinane structure, a tetrazinane structure, a pentazinane structure, and a quinuclidine structure, an oxygen-containing saturated ring such as a tetrahydropyran structure and a morpholine structure, a sulfur-containing saturated ring such as a tetrahydrothiopyran structure, and the like.

Examples of the 6-membered ring of a partially saturated ring include a hydrocarbon-based partially saturated ring such as a cyclohexene structure and a cyclohexadiene structure, a nitrogen-containing partially saturated ring such as a piperidine structure, an oxygen-containing partially saturated ring such as a pyran structure, a sulfur-containing partially saturated ring such as a thiazine structure, and the like.

Examples of the 6-membered ring of an aromatic ring include a hydrocarbon-based aromatic ring such as a benzene structure, a nitrogen-containing aromatic ring (nitrogen-containing unsaturated rings) such as a pyridine structure, a pyridazine structure, a pyrimidine structure, a pyrazine structure, a triazine structure, a tetrazine structure, and a pentazine structure, and the like.

Examples of the fused ring include a fused ring of a 6-membered ring and a 5-membered ring, a fused ring of two 6-membered rings, and the like. Among these, examples of the fused ring of a 6-membered ring and a 5-membered ring include an indole structure such as indole, indolenine, indoline, isoindole, isoindolenine, isoindoline, isodoridine, purine, and indolizidine. Examples of the fused ring of two 6-membered rings include quinoline structures such as quinoline, isoquinoline, quinolizidine, quinoxaline, cinnoline, quinazoline, phthalazine, naphthyridine, and pteridine.

In a case where the ring A is a 6-membered ring, R² to R⁶ are each independently a hydrogen atom, a hydroxyl group, an amino group, an alkoxy group, a carboxy group, or a carbonyl group. In addition, in a case where the ring A is a 5-membered ring, R² to R⁵ are each independently a hydrogen atom, a hydroxyl group, an amino group, an alkoxy group, a carboxy group, or a carbonyl group.

Note that in a case where any of R² to R⁶ when the ring A is a 6-membered ring, or any of R² to R⁵ when the ring A is a 5-membered ring is a carbonyl group, this indicates a structure in which a ring-constituting atom of the ring A is a carbon atom, and the carbon atom and an oxygen atom are linked via a double bond, and refers to a carbonyl group.

Specific examples of the cyclic carboxylic acid compound include various hydroxybenzoic acids such as 2-hydroxybenzoic acid (salicylic acid), 3-hydroxybenzoic acid (m-salicylic acid), 4-hydroxybenzoic acid (p-salicylic acid), 2,3-dihydroxybenzoic acid (pyrocatechic acid), 2,4-dihydroxybenzoic acid (β-resorcylic acid), 2,5-dihydroxybenzoic acid (gentisic acid), 2,6-dihydroxybenzoic acid (γ-resorcylic acid), 3,4-dihydroxybenzoic acid (protocatechuic acid), 3,5-dihydroxybenzoic acid (α-resorcylic acid), 3,4,5-trihydroxybenzoic acid (gallic acid), 4-hydroxy-3-methoxybenzoic acid (vanillic acid), and 3-hydroxy-4-methoxybenzoic acid (isovanillic acid), various aminobenzoic acids such as 2-aminobenzoic acid, 3-aminobenzoic acid, and 4-aminobenzoic acid, various shikimic acids such as dehydroshikimic acid and shikimic acid, methoxybenzoic acid, syringic acid, and the like.

Among these, the cyclic carboxylic acid compound is particularly preferably hydroxybenzoic acids, aminobenzoic acids, or shikimic acids. These can be easily prepared from a biomass-derived starting material.

In addition, the cyclic carboxylic acid compound is particularly preferably 4-hydroxybenzoic acid, 3,4-dihydroxybenzoic acid, 4-aminobenzoic acid, or shikimic acid. Through a decarboxylation reaction, these produce cyclic compounds that are particularly useful as starting materials for chemical products, fragrances, cosmetics, pharmaceuticals, agricultural chemicals, and the like.

A molecular weight of the cyclic carboxylic acid compound is not particularly limited, but is preferably 120 to 1,000, and more preferably 130 to 850.

The starting material containing the cyclic carboxylic acid compound may be a starting material derived from fossil resources, but is preferably a biomass-derived starting material. Biomass refers to plant-derived organic resources. Specific examples include plants that have been converted and stored in the form of starch, cellulose, and the like, animals that grow by eating plants, products made by processing plants or animals, and the like.

In order to prepare a starting material containing a cyclic carboxylic acid compound from biomass, pretreatment is first carried out. With this, mixed sugar such as oligosaccharides or polysaccharides having glucose units is obtained from the biomass.

In a case where the cyclic carboxylic acid compound is 4-hydroxybenzoic acid, for example, a starting material containing 4-hydroxybenzoic acid can be obtained from mixed sugar by the method disclosed in Biotechnology and Bioengineering Vol. 76, Section 376, 2001. Specifically, for example, 4-hydroxybenzoic acid can be obtained by fermentation using bacteria or recombinant bacteria and using mixed sugar such as glucose as a carbon source. Bacteria or recombinant bacteria used for fermentation are not particularly limited.

Note that crystallization fermentation is carried out using microorganisms that can grow in weak acidity near the acid dissociation constant (pKa) at which 4-hydroxybenzoic acid becomes insoluble, and a target 4-hydroxybenzoic acid may be obtained by solid-liquid separation.

In addition, by adding an amine solvent that is immiscible with water to an ammonium salt type 4-hydroxybenzoic acid aqueous solution obtained by fermentation and heating thereof, ammonia is removed and an amine solution of 4-hydroxybenzoic acid may be obtained.

Furthermore, 4-hydroxybenzoic acid is esterified with alcohol, an obtained 4-hydroxybenzoic acid ester is purified by distillation, then the 4-hydroxybenzoic acid ester is hydrolyzed, and 4-hydroxybenzoic acid may be produced.

On the other hand, for example, in a case where the cyclic carboxylic acid compound is 3,4-dihydroxybenzoic acid, a starting material containing 3,4-dihydroxybenzoic acid can be obtained by fermentation using recombinant bacteria and using mixed sugar such as glucose as a carbon source. The recombinant bacteria used for fermentation are not particularly limited.

Note that the present step may be a step of preparing a cyclic carboxylic acid compound produced by recycling and the like, for example.

As described above, in the present embodiment, it is preferable to use a biomass-derived starting material as the starting material containing a cyclic carboxylic acid compound. By including such a step, fossil resources are not consumed during the preparation of the cyclic carboxylic acid compound, and thus an increase in a concentration of carbon dioxide in the atmosphere can be suppressed. Therefore, this can contribute to suppression of global warming. Note that the starting material may be a mixture of a material derived from fossil resources and a material derived from biomass.

In addition, the starting material containing a cyclic carboxylic acid compound may or may not contain a solvent in addition to the cyclic carboxylic acid compound.

Among these, it is preferable that the starting material does not contain a solvent. In a case where no solvent is used, a reaction rate of the cyclic carboxylic acid compound in the heating step S02 described below can be increased. In addition, an amount of heat required for the heat treatment in the heating step S02 can be reduced. Therefore, production efficiency can be particularly improved. Note that "not containing a solvent" refers to a state in which a content of a solvent is 1% by mass or less of a total starting material.

On the other hand, in a case where the starting material contains a solvent, the fluidity of the starting material can be increased, and thus the handleability of the starting material can be favorable. However, in a case where an amount of the solvent is too large, an amount of heat required in the heating step S02 increases.

Therefore, in a case where the starting material contains a solvent, a content of the solvent is more than 1% by mass of the entire starting material, and a mass ratio of the solvent to the cyclic carboxylic acid compound is preferably 150 or less, and more preferably 15 or less.

Examples of the solvent include water, alcohol, alkylene glycol, aromatic hydrocarbons, ethers, and the like, and one alone or a mixture of two or more of these, or a mixture of at least one of these and other solvents is used.

Examples of the alcohol include methanol, ethanol, propanol, isopropyl alcohol, butanol, pentanol, hexanol, and the like.

Examples of the alkylene glycol include ethylene glycol, diethylene glycol, triethylene glycol, 1,2-propylene glycol, 1,3-propanediol, 1,4-butanediol, 1,5-pentanediol, 1,6-hexanediol, and the like.

Examples of aromatic hydrocarbons include benzene, toluene, xylene, phenol, cumene, mesitylene, naphthalene, tetraline, NMP (N-methyl-2-pyrrolidone), and the like.

Examples of the ethers include diphenyl ether, dimethyl ether, and the like.

### 2. Heating step S02

In the heating step S02, the starting material is subjected to a heat treatment of heating under a catalyst. With this, a decarboxylation reaction is caused in the cyclic carboxylic acid compound. As a result, a cyclic compound is obtained.

The decarboxylation reaction of the cyclic carboxylic acid compound is represented by the following formula.

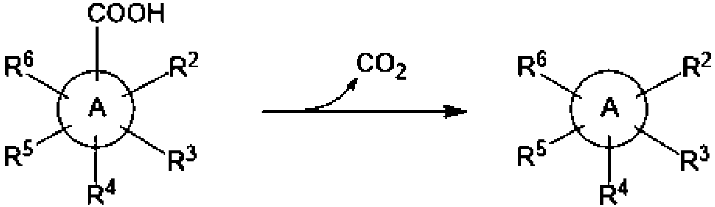

The above-described reaction formula is represented as follows in a case where the cyclic carboxylic acid compound is, for example, 4-hydroxybenzoic acid (p-salicylic acid).

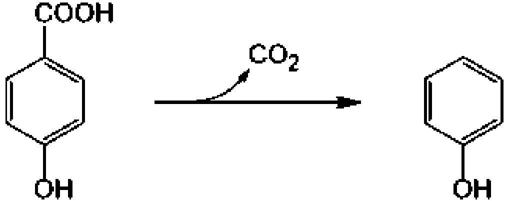

In addition, the above-described reaction formula is represented as follows in a case where the cyclic carboxylic acid compound is, for example, 3,4-dihydroxybenzoic acid (protocatechuic acid).

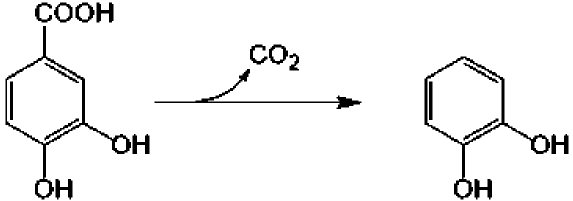

In addition, the above-described reaction formula is represented as follows in a case where the cyclic carboxylic acid compound is, for example, 4-aminobenzoic acid.

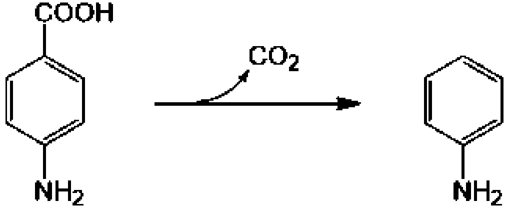

In addition, the above-described reaction formula is represented as follows in a case where the cyclic carboxylic acid compound is, for example, shikimic acid.

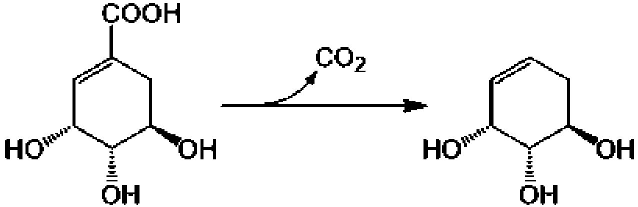

Specifically, the present step is carried out as follows.

First, a starting material containing a cyclic carboxylic acid compound is placed in a reactor. The reactor is not particularly limited as long as the reactor is made of a material that does not cause denaturation of the starting material. In addition, although the reactor has a function of adjusting temperature, it may also have a function of adjusting pressure.

Next, a catalyst is placed to the reactor. Then, a heat treatment is carried out to heat the inside of the reactor. As a result, the catalyst acts on the cyclic carboxylic acid compound contained in the starting material, and strong carbon-carbon bonds are broken. That is, the decarboxylation reaction of the cyclic carboxylic acid compound is promoted by the catalyst. With this, a cyclic compound can be obtained in a high yield. Note that the catalyst may be placed in the reactor first, or the starting material and the catalyst may be placed in the reactor at the same time.

A temperature in the heat treatment is preferably 150°C to 350°C, more preferably 180°C to 300°C, and further more preferably 200°C to 290°C. By carrying out the heat treatment at this temperature, the decarboxylation reaction can be further promoted. With this, the cyclic compound can be efficiently produced at a low cost.

Note that in a case where the heating temperature is less than a lower limit value, there is a concern that the promoting action of the catalyst on the decarboxylation reaction decreases, and the yield of the cyclic compound decreases. On the other hand, in a case where the heating temperature exceeds an upper limit value, the yield will hardly increase, but energy consumption increases, leading to an increase in production cost.

The heating temperature may be adjusted depending on a melting point of the cyclic carboxylic acid compound. The heating temperature is preferably higher than the melting point of the cyclic carboxylic acid compound, more preferably 10°C or higher than the melting point. This makes the reaction system more homogeneous, and thus the catalyst promotes the decarboxylation reaction more markedly. As a result, the yield of the cyclic compound can be further increased.

For example, since the melting point of 4-hydroxybenzoic acid is 214°C, the heating temperature in a case where the starting material containing 4-hydroxybenzoic acid is subjected to a heat treatment is preferably higher than 214°C and 350°C or less, and more preferably 224°C or higher and 290°C or lower.

In addition, since the melting point of 3,4-hydroxybenzoic acid is 221°C, the heating temperature in a case where the starting material containing 3,4-hydroxybenzoic acid is subjected to a heat treatment is preferably higher than 221°C and 350°C or lower, and more preferably 231°C or higher and 290°C or lower.

On the other hand, since the melting point of 4-aminobenzoic acid is 187°C, the heating temperature in a case where the starting material containing 4-aminobenzoic acid is subjected to a heat treatment is preferably higher than 187°C and 350°C or lower, and more preferably 197°C or higher and 290°C or lower.

In addition, a heating time at such a heating temperature is appropriately set in consideration of the reaction rate of the decarboxylation reaction and other reaction conditions, but as an example, the heating time is preferably 5 minutes or more and 24 hours or less, more preferably 10 minutes or more and 12 hours or less, and further more preferably 30 minutes or more and 6 hours or less.

A pressure inside the reactor during the heat treatment is not particularly limited, and may be normal pressure, less than normal pressure, or above normal pressure. Normal pressure refers to 86 kPa or more and 106 kPa or less. In a case where the pressure is normal pressure, the pressure resistance of the reactor can be lowered, and thus the reactor can be easily enlarged.

On the other hand, in a case where the pressure inside the reactor is lower than normal pressure, the volatility of the cyclic compound obtained after the decarboxylation reaction can be increased. With this, the cyclic compound can be purified by condensing the volatilized cyclic compound, and a cyclic compound with higher purity can be produced.

The specific pressure is preferably 0.01 atm (1 kPa) or more and less than normal pressure, and more preferably 0.1 atm or more and less than 0.7 atm (71 kPa). In addition, in a case where the pressure in the heat treatment is less than the lower limit value, there is a concern that the volatility of the cyclic carboxylic acid compound contained in the starting material also increases, and the yield decreases.

In addition, in a case where the pressure inside the reactor is made to exceed normal pressure, the temperature inside the reactor can be made to be equal to or higher than a boiling point of the solvent. With this, the decarboxylation reaction by the catalyst is more significantly promoted. As a result, the yield of the cyclic compound can be further increased.

The specific pressure is preferably higher than normal pressure and 10 MPa or less, and more preferably 200 kPa or more and 1 MPa or less. This eliminates the need to increase the pressure resistance of the reactor more than necessary, making it easier to reduce costs.

In addition, the atmosphere inside the reactor is not particularly limited, and may be, for example, an air atmosphere, an inert gas atmosphere, or the like. Among these, an inert gas atmosphere may be selected when suppressing side reactions such as oxidation of a cyclic compound. Examples of the inert gas include nitrogen, carbon dioxide, argon, and the like.

The reactor may be a batch reactor, a semi-batch reactor, a continuous reactor, or a flow reaction system reactor. Among these, the flow reaction system reactor is a reactor in which a starting material is inserted into one side of a reaction tube filled with a catalyst, and a product is taken out from the other side.

Moreover, since the cyclic carboxylic acid compound becomes a gas under heating conditions of the boiling point or higher of the cyclic carboxylic acid compound, the reaction mode is preferably a fixed bed type, a moving bed type, or a fluidized bed type. Among these, the fixed bed type is particularly preferable because the reactor structure is not complicated and the construction cost is relatively low. In the case of heating, a tube heating furnace type that heats from the outside can be used.

Furthermore, the reactor may have a structure that allows the starting material and the catalyst to be sealed, or may have a structure that does not seal the reactor.

In addition, after the cyclic compound is produced, a step of separating or purifying the cyclic compound may be provided depending on the necessity.

Next, the catalyst will be explained.

Constituent materials of the catalyst include zeolite. Zeolite is a material that is made of an aluminum/silicon-containing composite oxide and contains many pores. The aluminum/silicon-containing composite oxide refers to a substance in which aluminum oxide and silicon oxide are chemically composited.

The aluminum/silicon containing composite oxide is represented by General Formula (1).

Me_{2/X}O/Al₂O₃/mSiO₂/nH₂O (1)

In the formula (1), Me is an X-valent cation present in the pores of the zeolite, m is referred to as an SiO₂/Al₂O₃ ratio (silica alumina ratio) and takes a value of 2 or more. n represents an amount of adsorbed water.

The SiO₂/Al₂O₃ ratio of zeolite is 7 to 500. The SiO₂/Al₂O₃ ratio of zeolite is a ratio of a molar amount of SiO₂ to a molar amount of Al₂O₃ in the zeolite, and is an indicator that represents the acid strength and acid amount when the zeolite behaves as a solid acid.

The present inventors found that the SiO₂/Al₂O₃ ratio of zeolite is related to the performance of a catalyst used during producing a cyclic compound from a cyclic carboxylic acid compound. The present inventors also found that when the SiO₂/Al₂O₃ ratio is within the above range, the decarboxylation reaction in a cyclic carboxylic acid compound is particularly promoted by a catalyst, thereby completing the present invention.

Therefore, in a case where the SiO₂/Al₂O₃ ratio is within the above range, the efficiency of the decarboxylation reaction is particularly increased by the action of the catalyst, and the yield of the cyclic compound can be particularly increased. Note that in a case where the SiO₂/Al₂O₃ ratio is below the lower limit value, the acid strength of the solid acid decreases. Therefore, the performance of the catalyst deteriorates and the yield of the cyclic compound decreases. On the other hand, in a case where the SiO₂/Al₂O₃ ratio exceeds the upper limit value, the acid amount in the solid acid decreases. Therefore, the performance of the catalyst deteriorates and the yield of the cyclic compound decreases.

Note that the SiO₂/Al₂O₃ ratio can be obtained by, for example, fluorescent X-ray analysis and the like.

As described above, the method for producing a cyclic compound according to the present embodiment includes the above-described heating step S02. In the heating step S02, the starting material containing a cyclic carboxylic acid compound is subjected to a heat treatment of heating under a catalyst. With this, a decarboxylation reaction is caused in the cyclic carboxylic acid compound. As a result, a cyclic compound is obtained. The catalyst contains zeolite made of aluminum/silicon-containing composite oxide. The SiO₂/Al₂O₃ ratio of the zeolite is 7 to 500.

According to such a production method, the efficiency of the decarboxylation reaction is particularly increased by the action of the catalyst, and the yield of the cyclic compound can be particularly increased. Furthermore, the obtained cyclic compound exhibits high quality, for example, less coloring and the like.

In addition, the SiO₂/Al₂O₃ ratio of the zeolite is preferably 7 to 50, more preferably 10 to 45, and further more preferably 15 to 43.

The cyclic compound obtained through the present step is a compound obtained by removing the carboxy group of the cyclic carboxylic acid compound, as shown in the above-described reaction formula.

For example, in a case where the cyclic carboxylic acid compound is 4-hydroxybenzoic acid, the produced cyclic compound is phenol. In a case where the cyclic carboxylic acid compound is 3,4-dihydroxybenzoic acid, the produced cyclic compound is catechol. In a case where the cyclic carboxylic acid compound is 4-aminobenzoic acid, the produced cyclic compound is aniline.

Cyclic compounds produced from cyclic carboxylic acid compounds are particularly useful as starting materials for products such as chemical products, fragrances, cosmetics, pharmaceuticals, and agricultural chemicals. Among these, examples of the chemical products include materials for electrical/electronic parts, materials for synthetic fibers, resin materials, and chemical materials. Note that the starting materials include intermediates.

An acid amount of the zeolite contained in the catalyst determined by the NH₃-TPD method is preferably 0.1 to 2.5 [mmol/g], more preferably 0.4 to 2.2 [mmol/g], and further more preferably 0.8 to 1.9 [mmol/g]. An acid amount of the zeolite determined by the NH₃-TPD method affects the performance of the catalyst. Specifically, in a case where the acid amount is increased, the performance of the catalyst is improved and the yield of the cyclic compound can be increased. In addition, in a case where the acid amount of the zeolite determined by the NH₃-TPD method is within the above range, the purity of the cyclic compound can be increased, and thus, for example, coloring of the cyclic compound can be suppressed. With this, when producing various products using the cyclic compound as a starting material, the quality of the product can be improved. In other words, the quality of the cyclic compound can be improved. Furthermore, it is possible to omit the purification of the starting material and reduce the number of processes in the purification step, and thus contributes to reduction of the number of processes for producing the product.

The NH₃-TPD method is an evaluation method in which NH₃ gas is measured by adsorbing NH₃ gas onto a sample to be measured, and continuously raising a temperature to desorb NH₃ gas. The above-described acid amount is a value measured by the NH₃-TPD method according to "Measurement of solid acid properties by ammonia temperature-programmed desorption method, catalyst, vol. 42, p. 218 (2000)".

Specifically, the acid amount of the zeolite determined by the NH₃-TPD method is measured as follows.

First, 0.4 g of a sample is left standing under vacuum evacuation at 500°C for 1 hour, and this is used as pretreatment. A mixed gas containing 10% by volume of ammonia and 90% by volume of helium is passed through the sample after 1 hour of pretreatment at room temperature to carry out saturated adsorption of ammonia onto the sample. After flowing the mixed gas for 15 minutes, a temperature of the sample is raised up to 100°C while flowing helium gas instead of the mixed gas. After raising the temperature, vacuum evacuation is carried out at 100°C for 5 minutes to remove ammonia remaining in the atmosphere. After removing the remaining ammonia, the temperature is raised to up to 700°C at a temperature rising rate of 10°C/min while flowing helium gas at a flow rate of 60 mL/min. Then, using a TCD detector, an amount of ammonia measured during the temperature rising process is measured. From the measured ammonia amount, the adsorbed ammonia amount per unit mass of the sample is calculated, and this is defined as the acid amount (mmol/g).

The aluminum/silicon-containing composite oxide has crystallinity. The aluminum/silicon-containing composite oxide often contains regularly arranged pores derived from the crystal structure.

The skeleton structure of the zeolite contained in the catalyst is compiled into a database by the International Zeolite Society, and is expressed by a structural code consisting of three capital letters. Examples of the zeolite contained in the catalyst include LTA type zeolite, FER type zeolite, MWW type zeolite, MFI type zeolite, MOR type zeolite, LTL type zeolite, FAU type zeolite, BEA type zeolite, and the like.

Among these, the catalyst preferably contains at least one of FER type zeolite, MFI type zeolite, BEA type zeolite, and MOR type zeolite, and more preferably contains at least one of MFI type zeolite, BEA type zeolite, and MOR type zeolite.

A zeolite having such a skeleton structure has a structure in which pores having a diameter appropriate to the molecular size of the cyclic carboxylic acid compound are distributed. In other words, a zeolite having such a skeleton structure has a structure which has pores having a diameter (pore diameter) through which a cyclic carboxylic acid compound can easily enter, and has a sufficiently large number of pores. Therefore, the catalyst containing this zeolite greatly contributes to promoting the decarboxylation reaction and can particularly increase the yield of the cyclic compound.

In addition, this structural code includes one or two or more of crystal forms.

Examples of the crystal form of LTA type zeolite include A type and the like. Examples of the crystal form of FER type zeolite include ferrierite type and the like. Examples of the crystal form of MWW type zeolite include MCM-22 type and the like. Examples of the crystal form of MFI type zeolite include ZSM-5 type and the like. Examples of the crystal form of MOR type zeolite include mordenite type and the like. Examples of the crystal form of LTL type zeolite include L type and the like. Examples of crystal form of FAU type zeolite include X type, Y type, faujasite type, and the like. Examples of the crystal form of BEA type zeolite include beta type and the like.

In particular, the zeolite contained in the catalyst preferably contains at least one crystal form among ferrierite type, ZSM-5 type, beta type, and mordenite type, and more preferably contains at least one crystal form among ZSM-5 type, beta type, and mordenite type.

Zeolite containing such a crystal form particularly improves the performance of the catalyst, and thus has an excellent effect of producing a highly pure cyclic compound, and can suppress coloring of the cyclic compound, for example. With this, the zeolite has an effect capable of producing various products using the cyclic compound as a starting material, and easily improving the quality of the product.

As described above, the pore diameter of the zeolite is optimized depending on the molecular size of the cyclic carboxylic acid compound, but as an example, is preferably 0.4 nm or more and 1.5 nm or less, more preferably 0.5 nm or more and 1.0 nm or less, and further more preferably 0.5 nm or more and 0.8 nm or less. In a case where the pore diameter is within the above range, molecules of the cyclic carboxylic acid compound will easily enter into the pores. Therefore, the decarboxylation reaction caused within the pores is particularly promoted, and the yield of the cyclic compound can be particularly increased. In addition, examples of negative effects of having a too large pore diameter are that molecules larger than those of the cyclic carboxylic acid compound are allowed to pass through the pores, shape selectivity of the zeolite for the cyclic carboxylic acid becomes poor, and the yield of the cyclic compound is decreased.

Note that in a case where the pore diameter is less than the lower limit value, molecules of some types of cyclic carboxylic acid compounds will have difficulty in entering the pores, and there is a concern that promotion of the decarboxylation reaction by the catalyst is inhibited. On the other hand, in a case where the pore diameter exceeds the above upper limit value, there is a concern that even molecules larger than those of the cyclic carboxylic acid compound are allowed to pass through the pores, and thus the opportunity to promote the decarboxylation reaction is reduced.

In addition, in a case where the zeolite has a plurality of pores with different pore diameters, it is sufficient that at least one pore diameter is within the above range. Moreover, the pore diameter can be measured, for example, by a nitrogen adsorption method.

The cations compensate for the negative charges in the skeleton structure of the zeolite. Examples of the cations include hydrogen ions, potassium ions, sodium ions, ammonium ions, and the like. In other words, the zeolite preferably contains hydrogen, potassium, sodium, ammonium, and the like. Note that in addition to the above ones, the cation may be an organic cation (template).

Moreover, the zeolite particularly preferably contains hydrogen. The zeolite containing hydrogen behaves as a solid acid. The solid acid is particularly useful in a carbon-carbon bond cleavage reaction, and particularly promotes a decarboxylation reaction. With this, the yield of the cyclic compound can be particularly increased. In addition, in a case where the zeolite contains hydrogen, it is easy to increase the purity of the cyclic compound, and thus, for example, coloring of the cyclic compound can be suppressed.

A content of the zeolite in the catalyst is preferably 60% by mass or more and 100% by mass or less. With this, the zeolite is present dominantly in the catalyst, and thus the catalytic action of the zeolite is sufficiently exhibited. In addition to zeolite, various additives such as a binder, a forming aid, and a water-soluble sodium salt may be added to the catalyst.

Examples of the binder include clay, silica sol, alumina, and the like.

Examples of forming aids include cellulose, alcohol, lignin, starch, guar gum, and the like.

Examples of the water-soluble sodium salt include inorganic sodium salts such as sodium phosphate, sodium silicate, and sodium aluminate, organic sodium salts such as general organic carboxylic acid, aminocarbonate, ether carboxylate, and vinyl-type polymer sodium salt, and the like.

The form of the catalyst is not particularly limited, and may be, for example, a form such as powder, granules, and formed bodies. In particular, catalysts shaped to a certain size are preferably used as catalysts for fixed bed reactors. Examples of the shape of the shaped catalyst include spherical, tablet, pellet, ring, and honeycomb shapes, which are appropriately selected depending on the flow rate or pressure. In addition, different forms of catalysts may be used in combination.

An average particle diameter of the powdered or granular catalyst is preferably about 1 to 500 µm, and more preferably about 2 to 300 um. In a case where the average particle diameter of the catalyst is within the above range, a specific surface area of the catalyst becomes sufficiently large, and the handleability of the catalyst becomes relatively favorable.

Note that the average particle diameter of the catalyst is a value obtained by averaging particle diameters of 10 or more catalysts when the catalysts are observed under a microscope.

An addition amount of the catalysts to the starting material is not particularly limited, but is preferably an amount such that the amount of zeolite is 0.01 to 1.5 mol, and more preferably an amount such that the amount of zeolite is 0.10 to 1.0 mol per 1.0 mol of the cyclic carboxylic acid compound. With this, a necessary and sufficient amount of catalyst acts on the cyclic carboxylic acid compound. As a result, it is possible to sufficiently increase the yield of the cyclic compound, and to suppress the negative effect due to an excessive amount of the catalyst, for example, a defect such as a decrease in the separability of the cyclic compound.

In addition, in the case of a flow reaction system reactor, the W/F is preferably 0.01 to 10,000 [g·h/mol], and more preferably 0.1 to 1,000 [g·h/mol]. Note that W is a mass (g) of the catalyst filled in the reaction tube, and F is a flow rate (mol/h) of the cyclic carboxylic acid compound flowing to a layer filled with the catalyst. Therefore, W/F is a ratio of the mass of the filled catalyst to the flow rate of the cyclic carboxylic acid compound flowing into the reaction tube.

In addition, the specific surface area of the zeolite is preferably 100 to 1,000 [m²/g], and more preferably 200 to 600 [m²/g]. If the specific surface area of the zeolite is within the above range, it means that the zeolite has pores with a sufficient density. Therefore, the yield of the cyclic compound can be particularly increased.

Note that the specific surface area of zeolite is measured, for example, by the BET method.

### <Second embodiment>

Next, a second embodiment of the method for producing a cyclic compound of the present invention will be described.

Hereinafter, the second embodiment of the method for producing a cyclic compound of the present invention will be described, but the differences from the first embodiment will be mainly described, and the explanation on the same matters will be omitted.

The method for producing a cyclic compound of the present embodiment has at least a step of causing a decarboxylation reaction in the cyclic carboxylic acid compound by subjecting the starting material containing the cyclic carboxylic acid compound to a heat treatment of heating in the presence of a non-solid acid to obtain a cyclic compound. That is, the method for producing a cyclic compound of the present embodiment is the same as the above-described first embodiment, except that instead of subjecting a starting material containing a cyclic carboxylic acid compound to a heat treatment of heating under the above-described catalyst, the starting material containing a cyclic carboxylic acid compound is subjected to a heat treatment of heating in the presence of the non-solid acid.

Here, as an example, an embodiment having a starting material preparation step S01, which is an optional step, and the above-described heating step S02 will be described, but the starting material preparation step S01 of the present embodiment is the same as the starting material preparation step S01 of the first embodiment, and thus the explanation will be omitted.

### 2. Heating step S02

In the heating step S02, the starting material is subjected to a heat treatment of heating in the presence of a non-solid acid. With this, a decarboxylation reaction is caused in the cyclic carboxylic acid compound. As a result, a cyclic compound is obtained.

Note that the decarboxylation reaction of the cyclic carboxylic acid compound is as shown in the above-described first embodiment.

Specifically, the present step is carried out as follows.

First, a starting material containing a cyclic carboxylic acid compound is placed in a reactor. The reactor is not particularly limited as long as the reactor is made of a material that does not cause denaturation of the starting material. In addition, although the reactor has a function of adjusting temperature, it may also have a function of adjusting pressure.

Next, a non-solid acid is placed in the reactor. Then, a heat treatment is carried out to heat the inside of the reactor. With this, the non-solid acid acts on the cyclic carboxylic acid compound contained in the starting material, and strong carbon-carbon bonds are broken. That is, the decarboxylation reaction of the cyclic carboxylic acid compound is promoted by the non-solid acid. With this, a cyclic compound can be obtained in a high yield. Note that the non-solid acid may be placed in the reactor first, or the starting material and the non-solid acid may be placed at the same time. In addition, the starting material may contain a non-solid acid together with the cyclic carboxylic acid compound.

A temperature in the heat treatment is preferably 150°C to 350°C, more preferably 180°C to 300°C, and further more preferably 200°C to 290°C. By carrying out the heat treatment at this temperature, the decarboxylation reaction can be further promoted. With this, the cyclic compound can be efficiently produced at a low cost.

Note that in a case where the heating temperature is less than the lower limit value, there is a concern that the promoting action of the non-solid acid on the decarboxylation reaction decreases, and the yield of the cyclic compound decreases. On the other hand, in a case where the heating temperature exceeds an upper limit value, the yield will hardly increase, but energy consumption increases, leading to an increase in production cost.

The heating temperature may be adjusted depending on a melting point of the cyclic carboxylic acid compound. The heating temperature is preferably higher than the melting point of the cyclic carboxylic acid compound, more preferably 10°C or higher than the melting point. With this, since the reaction system becomes more homogeneous, the non-solid acid promotes the decarboxylation reaction more markedly. As a result, the yield of the cyclic compound can be further increased.

For example, since the melting point of 4-hydroxybenzoic acid is 214°C, the heating temperature in a case where the starting material containing 4-hydroxybenzoic acid is subjected to a heat treatment is preferably higher than 214°C and 350°C or less, and more preferably 224°C or higher and 290°C or lower.

In addition, since the melting point of 3,4-hydroxybenzoic acid is 221°C, the heating temperature in a case where the starting material containing 3,4-hydroxybenzoic acid is subjected to a heat treatment is preferably higher than 221°C and 350°C or lower, and more preferably 231°C or higher and 290°C or lower.

On the other hand, since the melting point of 4-aminobenzoic acid is 187°C, the heating temperature in a case where the starting material containing 4-aminobenzoic acid is subjected to a heat treatment is preferably higher than 187°C and 350°C or lower, and more preferably 197°C or higher and 290°C or lower.

In addition, a heating time at such a heating temperature is appropriately set in consideration of the reaction rate of the decarboxylation reaction and other reaction conditions, but as an example, the heating time is preferably 5 minutes or more and 24 hours or less, more preferably 10 minutes or more and 12 hours or less, and further more preferably 30 minutes or more and 6 hours or less.

A pressure inside the reactor during the heat treatment is not particularly limited, and may be normal pressure, less than normal pressure, or above normal pressure. Normal pressure refers to 86 kPa or more and 106 kPa or less. In a case where the pressure is normal pressure, the pressure resistance of the reactor can be lowered, and thus the reactor can be easily enlarged.

On the other hand, in a case where the pressure inside the reactor is lower than normal pressure, the volatility of the cyclic compound obtained after the decarboxylation reaction can be increased. With this, the cyclic compound can be purified by condensing the volatilized cyclic compound, and a cyclic compound with higher purity can be produced.

The specific pressure is preferably 0.01 atm (1 kPa) or more and less than normal pressure, and more preferably 0.1 atm or more and less than 0.7 atm (71 kPa). In addition, in a case where the pressure in the heat treatment is less than the lower limit value, there is a concern that the volatility of the cyclic carboxylic acid compound contained in the starting material also increases, and the yield decreases.

On the other hand, in a case where the pressure inside the reactor is set above normal pressure, the temperature inside the reactor can be raised to a temperature higher than a boiling point of the solvent. With this, the non-solid acid promotes the decarboxylation reaction more markedly. As a result, the yield of the cyclic compound can be further increased.

A specific pressure is preferably higher than normal pressure and 40 MPa or less, and more preferably 200 kPa or more and 10 MPa or less. This eliminates the need to increase the pressure resistance of the reactor more than necessary, making it easier to reduce costs.

In addition, the atmosphere inside the reactor is not particularly limited, and may be, for example, an air atmosphere, an inert gas atmosphere, or the like. Among these, an inert gas atmosphere may be selected when suppressing side reactions such as oxidation of a cyclic compound. Examples of the inert gas include nitrogen, carbon dioxide, argon, and the like.

The reactor may be a batch reactor, a semi-batch reactor, a continuous reactor, or a flow reaction system reactor.

Moreover, since the cyclic carboxylic acid compound becomes a gas under heating conditions of the boiling point or higher of the cyclic carboxylic acid compound, the reaction mode is preferably a fixed bed type, a moving bed type, or a fluidized bed type. Among these, the fixed bed type is particularly preferable because the reactor structure is not complicated and the construction cost is relatively low. In the case of heating, a tube heating furnace type that heats from the outside can be used.

In addition, the reactor may have a structure that allows the starting material and the non-solid acid to be sealed, or may have a structure that does not seal the starting material and the non-solid acid.

In addition, after the cyclic compound is produced, a step of separating or purifying the cyclic compound may be provided depending on the necessity.

Next, the non-solid acid will be explained. As described above, in the heating step S02, the starting material is subjected to a heat treatment of heating in the presence of a non-solid acid.

The non-solid acid used in the heating step S02 is an acid that is present as a liquid or gas during the heat treatment. In the case of a non-solid acid, the non-solid acid can be homogeneously mixed with the starting material when heated in the reactor. Therefore, the efficiency of contact between the starting material and the non-solid acid increases. Examples of the non-solid acid used in the heating step S02 include inorganic acids, organic acids, and the like.

Examples of the inorganic acid include sulfuric acid, carbonic acid, phosphoric acid, nitric acid, hydrochloric acid, and the like, and one or a mixture of two or more of these may be used.

In a case where a material containing an inorganic acid is used as the non-solid acid used in the heating step S02, since the inorganic acid is easily dissolved in water, the solubility of the non-solid acid in water can be easily increased. With this, in a case where the non-solid acid is used as an aqueous solution, a concentration of the non-solid acid can be easily adjusted. Therefore, the acid amount relative to the amount of cyclic carboxylic acid compound can be easily adjusted. As a result, excess or deficiency of the non-solid acid is less likely to occur, and the yield of the cyclic compound can be easily increased while suppressing the occurrence of a surplus of the non-solid acid.

In addition, many inorganic acids have a higher boiling point than organic acids. Therefore, when the produced cyclic compound is separated by evaporation separation and the like, the difference in boiling point between the cyclic compound and the non-solid acid can be increased. With this, the separability of the cyclic compound becomes favorable, and the yield and purity can be easily increased.

On the other hand, in a case where a material containing an organic acid is used as the non-solid acid used in the heating step S02, the strength of the organic acid as an acid is appropriate, and thus the load on the equipment can be reduced. Specifically, organic acids are less likely to destabilize a passive film of metal, and therefore less likely to cause corrosion of the metal. Therefore, it is not necessary to take special anticorrosion measures for the equipment, and the cost of the equipment can be reduced.

Furthermore, organic acids have moderate strength as acids compared to inorganic acids, and therefore are less likely to produce by-products. By-products cause coloring of the produced cyclic compound or cause a decrease in purity. Therefore, by suppressing by-products, it is possible to improve the quality of the cyclic compound. Note that a colored cyclic compound can be decolorized by additional purification treatment.

Examples of the organic acids include various carboxylic acids such as aliphatic carboxylic acids (fatty acids) and the derivatives thereof, various sulfonic acids and the derivatives thereof, and the like, and one or a mixture of two or more of these is used. In addition, various carboxylic acids include monocarboxylic acids, dicarboxylic acids, and tricarboxylic acids. Various sulfonic acids include monosulfonic acids, disulfonic acids, and trisulfonic acids. Examples of derivatives include esters, amides, acid anhydrides, acid chlorides, and the like.

Among the above ones, the organic acid used in the heating step S02 is preferably an aliphatic carboxylic acid or a derivative thereof. Aliphatic carboxylic acids or derivatives thereof are particularly unlikely to produce by-products during heat treatment. Therefore, aliphatic carboxylic acids or derivatives thereof are useful as organic acids used in the heating step S02.

Examples of aliphatic carboxylic acids include saturated aliphatic monocarboxylic acids such as formic acid, acetic acid, propionic acid, butyric acid, isobutyric acid, valeric acid, isovaleric acid, pivalic acid, caproic acid, glycolic acid, lactic acid, gluconic acid, and pyruvic acid, unsaturated aliphatic monocarboxylic acids such as acrylic acid, methacrylic acid, oleic acid, linoleic acid, and α-linolenic acid, saturated aliphatic dicarboxylic acids such as oxalic acid, malonic acid, succinic acid, malic acid, tartaric acid, α-ketoglutaric acid, glutaric acid, 2-oxoglutaric acid, and adipic acid, unsaturated aliphatic dicarboxylic acids such as maleic acid and fumaric acid, and tricarboxylic acids such as citric acid, isocitric acid, and aconitic acid.

In a case where the organic acid used in the heating step S02 is an aliphatic carboxylic acid, the number of carbon atoms in the aliphatic carboxylic acid is not particularly limited, but is preferably 1 or more and 8 or less, more preferably 2 or more and 6 or less, and further more preferably 3 or more and 5 or less. The aliphatic carboxylic acid having such a number of carbon atoms has a melting point at which the aliphatic carboxylic acid becomes a liquid or a boiling point at which the aliphatic carboxylic acid becomes a gas at the above-described heating temperature. Therefore, by performing a heat treatment in the presence of the aliphatic carboxylic acid having the above-described number of carbon atoms, a reaction system in which the aliphatic carboxylic acid is homogeneously mixed with the starting material can be created. In such a reaction system, the aliphatic carboxylic acid that acts like a catalyst has many opportunities to come into contact with the cyclic carboxylic acid compound. Therefore, the decarboxylation reaction of the cyclic carboxylic acid compound can be efficiently promoted by the aliphatic carboxylic acid.

Based on the above, the melting point of the non-solid acid is preferably 350°C or lower, and more preferably 300°C or lower. With this, the non-solid acid becomes a liquid or gas during the heat treatment, and thus the reaction system between the starting material and the non-solid acid becomes more homogeneous. As a result, the decarboxylation reaction can be particularly promoted.

In addition, the boiling point of the non-solid acid is preferably 80°C or higher, more preferably 90°C or higher and 350°C or lower, and further more preferably 100°C or higher and 250°C or lower. With this, the probability that the non-solid acid becomes a gas during the heat treatment is increased. As a result, non-solid acid can act particularly like a homogeneous catalyst, and particularly promote decarboxylation reactions.

In addition, in a case where the cyclic compound to be produced is purified and recovered by evaporation separation, the boiling point of the non-solid acid is preferably different from the boiling point of the cyclic compound to be produced. Specifically, when an absolute value of the difference between the boiling point of the non-solid acid and the boiling point of the cyclic compound is defined as the "boiling point difference", the boiling point difference is preferably greater than 0°C, more preferably 10°C to 250°C, and further more preferably 30°C to 200°C. In a case where the boiling point difference is within the above range, the separability of the cyclic compound in evaporation separation can be further improved. That is, when the cyclic compound is evaporated and recovered as the temperature rises, it is possible to suppress the non-solid acid from being mixed into the cyclic compound as an impurity.

Note that even in a case where the boiling point difference is outside the above range, the cyclic compound can be purified and recovered by adding another separation treatment.

In addition, among the aliphatic carboxylic acids, saturated aliphatic monocarboxylic acids and saturated aliphatic dicarboxylic acids are preferably used. These are also referred to as saturated fatty acids and are chemically stable. Therefore, decomposition, denaturation, or the like is less likely to occur during a heat treatment. Therefore, it is easy to increase the purity of the cyclic compound obtained by the decarboxylation reaction.

On the other hand, the strength of a non-solid acid is represented by the acid dissociation constant pKa. The acid dissociation constant pKa of the non-solid acid used in the heating step S02 is not particularly limited, but is preferably 5.00 or less, more preferably -5.00 or more and 4.80 or less, and further more preferably -4.00 or more and 4.50 or less. In a case where the acid dissociation constant pKa is within the above range, the action of the non-solid acid becomes particularly large. With this, the yield of the cyclic compound can be particularly increased. Note that the acid dissociation constant pKa of the non-solid acids is a value for an aqueous solution of each non-solid acid at 25°C. In a case where a non-solid acid has two or more acidic hydrogens, acid dissociation occurs in stages and has a plurality of acid dissociation constants pKa. In this case, the lowest acid dissociation constant is used.

As described above, the method for producing a cyclic compound according to the present embodiment has a heating step S02. In the heating step S02, the starting material containing a cyclic carboxylic acid compound is subjected to a heat treatment of heating in the presence of a non-solid acid. With this, a decarboxylation reaction is caused in the cyclic carboxylic acid compound. As a result, a cyclic compound is obtained.

According to such a production method, the efficiency of the decarboxylation reaction is particularly increased by the action of the non-solid acid, and the yield of the cyclic compound can be particularly increased. In addition, the non-solid acid used in the heating step S02 is present as a liquid or gas during the heat treatment and is homogeneously mixed with the cyclic carboxylic acid compound contained in the starting material, and thus can act like a so-called homogeneous catalyst. With this, the reaction efficiency of the decarboxylation reaction can be easily increased without using subcritical water in the related art. With this, it is possible to easily reduce the cost of equipment used for producing the cyclic compound.

An addition amount of the non-solid acid to the cyclic carboxylic acid compound is not particularly limited, but an addition concentration of the non-solid acid to a total of the cyclic carboxylic acid compound and the non-solid acid is preferably 0.1 to 10.0 [mol%], more preferably 0.4 to 5.0 [mol%], and further more preferably 0.7 to 3.0 [mol%]. With this, a necessary and sufficient amount of non-solid acid acts on the cyclic carboxylic acid compound. As a result, it is possible to sufficiently increase the yield of the cyclic compound, and to suppress the negative effect due to an excessive amount of the non-solid acid, for example, a defect such as a decrease in the separability of the cyclic compound.

The cyclic compound obtained through the heating step S02 is a compound from which the carboxy group of the cyclic carboxylic acid compound is removed, as shown in the above-described reaction formula.

For example, in a case where the cyclic carboxylic acid compound is 4-hydroxybenzoic acid, the produced cyclic compound is phenol. In a case where the cyclic carboxylic acid compound is 3,4-dihydroxybenzoic acid, the produced cyclic compound is catechol. In a case where the cyclic carboxylic acid compound is 4-aminobenzoic acid, the produced cyclic compound is aniline.

Such cyclic compounds are particularly useful as starting materials for products such as chemical products, fragrances, cosmetics, pharmaceuticals, and agricultural chemicals. Among these, examples of the chemical products include materials for electrical/electronic parts, materials for synthetic fibers, resin materials, and chemical materials. The starting materials include intermediates.

Note that the non-solid acid used in the heating step S02 may include both an inorganic acid and an organic acid. In this case, while performing the heating step S02, there may be a time when the inorganic acid and the organic acid are simultaneously present in the reactor, or there may be a time when the inorganic acid and the organic acid are separately present.

In addition, the heat treatment may be further carried out in the presence of a solid acid. That is, in the heating step S02, a non-solid acid and a solid acid may be used together. A solid acid is a solid that has acidic points on a surface. The solid acid has a function of donating protons to reactants and receiving electron pairs from the reactants. Similar to the non-solid acid, such a solid acid also promotes the decarboxylation reaction of a cyclic carboxylic acid compound.

In a case where a non-solid acid and a solid acid are used together, there may be a time when the non-solid acid and the solid acid are simultaneously present in the reactor for a period of time, or there may be a time when the non-solid acid and the solid acid are separately present for a period of time.

Examples of solid acids include zeolite, clay mineral, cation exchange resin, activated carbon, metal oxide, metal sulfide, metal salt, composite oxide, heteropolyacid, hydrated oxide, oxygen acid-supported metal oxide, and the like, and one is used alone or two or more of these are used in combination.

Among these, zeolite is preferably used as the solid acid. Zeolite is a material that is made of an aluminum/silicon-containing composite oxide and contains many pores. The aluminum/silicon-containing composite oxide refers to a substance in which aluminum oxide and silicon oxide are chemically composited. Zeolite has excellent durability and is easy to reuse, and thus is useful as a solid acid.

The aluminum/silicon containing composite oxide is represented by General Formula (1).

Me_{2/X}O/Al₂O₃/mSiO₂/nH₂O (1)

In the formula (1), Me is an X-valent cation present in the pores of the zeolite, m is referred to as an SiO₂/Al₂O₃ ratio (silica alumina ratio) and takes a value of 2 or more. n represents an amount of adsorbed water.

The SiO₂/Al₂O₃ ratio of the zeolite is preferably 7 to 500, more preferably 7 to 50, further more preferably 10 to 45, and particularly preferably 15 to 43. The SiO₂/Al₂O₃ ratio of zeolite is a ratio of a molar amount of SiO₂ to a molar amount of Al₂O₃ in the zeolite, and is an indicator that represents the acid strength and acid amount when the zeolite behaves as a solid acid. In a case where the SiO₂/Al₂O₃ ratio is within the above range, the efficiency of the decarboxylation reaction is particularly increased by the action of the solid acid, and the yield of the cyclic compound can be particularly increased.

Note that the SiO₂/Al₂O₃ ratio can be obtained by, for example, fluorescent X-ray analysis and the like.

The acid amount of the zeolite contained in the solid acid measured by the NH₃-TPD method is preferably 0.1 to 2.5 [mmol/g], more preferably 0.4 to 2.2 [mmol/g], and further more preferably 0.8 to 1.9 [mmol/g]. The acid amount of the zeolite determined by the NH₃-TPD method affects the performance of the solid acid. Specifically, in a case where the acid amount is increased, the performance of the solid acid is improved and the yield of the cyclic compound can be increased. In addition, in a case where the acid amount of the zeolite determined by the NH₃-TPD method is within the above range, the purity of the cyclic compound can be increased, and thus, for example, coloring of the cyclic compound can be suppressed. With this, when producing various products using the cyclic compound as a starting material, the quality of the product can be improved. In other words, the quality of the cyclic compound can be improved. Furthermore, it is possible to omit the purification of the starting material and reduce the number of processes in the purification step, and thus contributes to reduction of the number of processes for producing the product.

The NH₃-TPD method is an evaluation method in which NH₃ gas is measured by adsorbing NH₃ gas onto a sample to be measured, and continuously raising a temperature to desorb NH₃ gas. The above-described acid amount is a value measured by the NH₃-TPD method according to "Measurement of solid acid properties by ammonia temperature-programmed desorption method, solid acid, vol. 42, p. 218 (2000)".

Specifically, the acid amount of the zeolite determined by the NH₃-TPD method is measured as follows.

First, 0.4 g of a sample is left standing under vacuum evacuation at 500°C for 1 hour, and this is used as pretreatment. A mixed gas containing 10% by volume of ammonia and 90% by volume of helium is passed through the sample after 1 hour of pretreatment at room temperature to carry out saturated adsorption of ammonia onto the sample. After flowing the mixed gas for 15 minutes, a temperature of the sample is raised up to 100°C while flowing helium gas instead of the mixed gas. After raising the temperature, vacuum evacuation is carried out at 100°C for 5 minutes to remove ammonia remaining in the atmosphere. After removing the remaining ammonia, the temperature is raised to up to 700°C at a temperature rising rate of 10°C/min while flowing helium gas at a flow rate of 60 mL/min. Then, using a TCD detector, an amount of ammonia measured during the temperature rising process is measured. From the measured ammonia amount, the adsorbed ammonia amount per unit mass of the sample is calculated, and this is defined as the acid amount (mmol/g).

The aluminum/silicon-containing composite oxide has crystallinity. The aluminum/silicon-containing composite oxide often contains regularly arranged pores derived from the crystal structure.

A skeleton structure of the zeolite contained in the solid acid is compiled into a database by the International Zeolite Society, and is expressed by a structural code consisting of three capital letters. Examples of the zeolite contained in the solid acid include LTA type zeolite, FER type zeolite, MWW type zeolite, MFI type zeolite, MOR type zeolite, LTL type zeolite, FAU type zeolite, BEA type zeolite, and the like.

Among these, the solid acid preferably includes at least one of FER type zeolite, MFI type zeolite, BEA type zeolite, and MOR type zeolite, and more preferably includes at least one of MFI type zeolite, BEA type zeolite, and MOR type zeolite.

A zeolite having such a skeleton structure has a structure in which pores having a diameter appropriate to the molecular size of the cyclic carboxylic acid compound are distributed. In other words, a zeolite having such a skeleton structure has a structure which has pores having a diameter (pore diameter) through which a cyclic carboxylic acid compound can easily enter, and has a sufficiently large number of pores. Therefore, the solid acid containing this zeolite greatly contributes to promoting the decarboxylation reaction and can particularly increase the yield of the cyclic compound.

In addition, this structural code includes one or two or more of crystal forms.

Examples of the crystal form of LTA type zeolite include A type and the like. Examples of the crystal form of FER type zeolite include ferrierite type and the like. Examples of the crystal form of MWW type zeolite include MCM-22 type and the like. Examples of the crystal form of MFI type zeolite include ZSM-5 type and the like. Examples of the crystal form of MOR type zeolite include mordenite type and the like. Examples of the crystal form of LTL type zeolite include L type and the like. Examples of crystal form of FAU type zeolite include X type, Y type, faujasite type, and the like. Examples of the crystal form of BEA type zeolite include beta type and the like.

In particular, the zeolite contained in the solid acid preferably includes at least one crystal form among the ferrierite type, the ZSM-5 type, the beta type, and the mordenite type, and more preferably includes at least one crystal form of the ZSM-5 type, the beta type, and the mordenite type.

Zeolite containing such crystal forms particularly improves the performance of the solid acid, and thus is excellent in producing the cyclic compound with high purity. Therefore, coloring of the cyclic compound, for example, can be suppressed. With this, the zeolite has an effect capable of producing various products using the cyclic compound as a starting material, and easily improving the quality of the product.

As described above, the pore diameter of the zeolite is optimized depending on the molecular size of the cyclic carboxylic acid compound, but as an example, is preferably 0.4 nm or more and 1.5 nm or less, more preferably 0.5 nm or more and 1.0 nm or less, and further more preferably 0.5 nm or more and 0.8 nm or less. In a case where the pore diameter is within the above range, molecules of the cyclic carboxylic acid compound will easily enter into the pores. Therefore, the decarboxylation reaction caused within the pores is particularly promoted, and the yield of the cyclic compound can be particularly increased. In addition, examples of negative effects of having a too large pore diameter are that molecules larger than those of the cyclic carboxylic acid compound are allowed to pass through the pores, shape selectivity of the zeolite for the cyclic carboxylic acid becomes poor, and the yield of the cyclic compound is decreased.

Note that there is a concern that in a case where the pore diameter is below the lower limit value, molecules of some types of cyclic carboxylic acid compounds will have difficulty in entering the pores, and the promotion of the decarboxylation reaction by the solid acid is inhibited. On the other hand, in a case where the pore diameter exceeds the above upper limit value, there is a concern that even molecules larger than those of the cyclic carboxylic acid compound are allowed to pass through the pores, and thus the opportunity to promote the decarboxylation reaction is reduced.

In addition, in a case where the zeolite has a plurality of pores with different pore diameters, it is sufficient that at least one pore diameter is within the above range. Moreover, the pore diameter can be measured, for example, by a nitrogen adsorption method.

The cations compensate for the negative charges in the skeleton structure of the zeolite. Examples of the cations include hydrogen ions, potassium ions, sodium ions, ammonium ions, and the like. In other words, the zeolite preferably contains hydrogen, potassium, sodium, ammonium, and the like. Note that in addition to the above ones, the cation may be an organic cation (template).

Moreover, the zeolite particularly preferably contains hydrogen. The zeolite containing hydrogen behaves particularly favorably as a solid acid and can particularly increase the yield of the cyclic compound. In addition, in a case where the zeolite contains hydrogen, it is easy to increase the purity of the cyclic compound, and thus, for example, coloring of the cyclic compound can be suppressed.

A content of zeolite in the solid acid is preferably 60% by mass or more and 100% by mass or less. With this, the zeolite is present dominantly in the solid acid, and thus the action of the zeolite is sufficiently exhibited. In addition to zeolite, various additives such as a binder, a forming aid, and a water-soluble sodium salt may be added to the solid acid.

Examples of the binder include clay, silica sol, alumina, and the like.

Examples of forming aids include cellulose, alcohol, lignin, starch, guar gum, and the like.

Examples of the water-soluble sodium salt include inorganic sodium salts such as sodium phosphate, sodium silicate, and sodium aluminate, organic sodium salts such as general organic carboxylic acid, aminocarbonate, ether carboxylate, and vinyl-type polymer sodium salt, and the like.

The form of the solid acid is not particularly limited, and may be, for example, a form of powder, granules, formed bodies, or the like. In particular, solid acids shaped to a certain size are preferably used as solid acids for fixed bed reactors. Examples of the shape of the shaped solid acid include spherical, tablet, pellet, ring, and honeycomb shapes, which are appropriately selected depending on the flow rate and pressure. In addition, different forms of solid acids may be used in combination.

An average particle diameter of the solid acid in powder or granule form is preferably about 1 to 500 µm, and more preferably about 2 to 300 um. In a case where the average particle diameter of the solid acid is within the above range, the specific surface area of the solid acid becomes sufficiently large and the handleability of the solid acid becomes relatively favorable.

Note that the average particle diameter of the solid acid is a value obtained by averaging the particle diameters of 10 or more solid acids when the solid acid is observed under a microscope.

An addition amount of solid acid to the starting material is not particularly limited, but is preferably an amount such that the amount of the solid acid is 0.01 to 1.5 mol, and more preferably an amount such that the amount of the solid acid is 0.10 to 1.0 mol per 1.0 mol of the cyclic carboxylic acid compound. With this, a necessary and sufficient amount of solid acid acts on the cyclic carboxylic acid compound. As a result, it is possible to sufficiently increase the yield of the cyclic compound and to suppress the negative effect due to an excessive amount of the solid acid, for example, a defect such as a decrease in the separability of the cyclic compound.

In addition, in the case of a flow reaction system reactor, the W/F is preferably 0.01 to 10,000 [g·h/mol], and more preferably 0.1 to 1,000 [g·h/mol]. Note that W is the mass (g) of the solid acid filled in the reaction tube, and F is the flow rate (mol/h) of the cyclic carboxylic acid compound flowing to a layer filled with the solid acid. Therefore, W/F is a ratio of the mass of the filled solid acid to the flow rate of the cyclic carboxylic acid compound flowing into the reaction tube.

In addition, the specific surface area of the zeolite is preferably 100 to 1,000 [m²/g], and more preferably 200 to 600 [m²/g]. If the specific surface area of the zeolite is within the above range, it means that the zeolite has pores with a sufficient density. Therefore, the yield of the cyclic compound can be particularly increased. Note that the specific surface area of zeolite is measured, for example, by the BET method.

### <Third embodiment>

Next, a third embodiment of the cyclic compound of the present invention and the method for producing a cyclic compound of the present invention will be described.

Hereinafter, the third embodiment of the cyclic compound of the present invention and the method for producing a cyclic compound of the present invention will be described below, but the differences from the first and second embodiments will be mainly described, and the explanation on the same matters will be omitted.

### (Method for producing cyclic compound)

The method for producing a cyclic compound according to the present embodiment has at least a step of causing a decarboxylation reaction in the cyclic carboxylic acid compound by subjecting a starting material containing the cyclic carboxylic acid compound derived from biomass to a heat treatment to obtain a cyclic compound. Here, as an example, an embodiment including a starting material preparation step S01 and a heating step S02, which are optional steps, will be described.

### 1. Starting material preparation step S01

In the starting material preparation step S01, a starting material containing a cyclic carboxylic acid compound derived from biomass is prepared. The present step may be carried out depending on the necessity, and can be omitted in a case where a product has already been prepared.

Note that the cyclic carboxylic acid compound is a compound described in the above-described first embodiment.

Here, the starting material containing a cyclic carboxylic acid compound is a biomass-derived starting material. Biomass refers to plant-derived organic resources. Specific examples include plants that have been converted and stored in the form of starch, cellulose, and the like, animals that grow by eating plants, products made by processing plants or animals, and the like. By using biomass, fossil resources are not consumed during the preparation of cyclic carboxylic acid compounds, and thus it is possible to suppress an increase in the concentration of carbon dioxide in the atmosphere. Therefore, this can contribute to carbon neutrality. Note that the starting material may be a mixture of a material derived from fossil resources and a material derived from biomass.

Carbon in biomass contains radioactive carbon ¹⁴C to the same extent as in the environment. In contrast, carbon in fossil resources contains almost no radioactive carbon ¹⁴C. Therefore, by producing a cyclic compound using a biomass-derived starting material, a cyclic compound containing radioactive carbon ¹⁴C can be produced. Such a cyclic compound exhibits an effect of suppressing an increase in carbon dioxide in the atmosphere at the time of disposal, and therefore can contribute to carbon neutrality. Note that the cyclic compound containing radioactive carbon ¹⁴C will be described in detail later.

To prepare a starting material containing a cyclic carboxylic acid compound from biomass, first, the biomass is pretreated. With this, mixed sugar such as oligosaccharides or polysaccharides having glucose units is obtained from the biomass.

In a case where the cyclic carboxylic acid compound is 4-hydroxybenzoic acid, for example, a starting material containing 4-hydroxybenzoic acid can be obtained from mixed sugar by the method disclosed in Biotechnology and Bioengineering Vol. 76, Section 376, 2001. Specifically, 4-hydroxybenzoic acid is obtained, for example, by fermentation using bacteria or recombinant bacteria and using a mixed sugar such as glucose as a carbon source. Bacteria or recombinant bacteria used for fermentation are not particularly limited.

Note that crystallization fermentation is carried out using microorganisms that can grow in weak acidity near the acid dissociation constant (pKa) at which 4-hydroxybenzoic acid becomes insoluble, and a target 4-hydroxybenzoic acid may be obtained by solid-liquid separation.

In addition, by adding an amine solvent that is immiscible with water to an ammonium salt type 4-hydroxybenzoic acid aqueous solution obtained by fermentation and heating thereof, ammonia is removed and an amine solution of 4-hydroxybenzoic acid may be obtained.

Furthermore, 4-hydroxybenzoic acid is esterified with alcohol, an obtained 4-hydroxybenzoic acid ester is purified by distillation, then the 4-hydroxybenzoic acid ester is hydrolyzed, and 4-hydroxybenzoic acid may be produced.

On the other hand, for example, in a case where the cyclic carboxylic acid compound is 3,4-dihydroxybenzoic acid, a starting material containing 3,4-dihydroxybenzoic acid is obtained by fermentation using recombinant bacteria and using a mixed sugar such as glucose as a carbon source. The recombinant bacteria used for fermentation are not particularly limited.

Note that the present step may be a step of preparing a biomass-derived starting material produced by recycling and the like, for example.

In addition, the starting material containing a cyclic carboxylic acid compound may or may not contain a solvent in addition to the cyclic carboxylic acid compound.

Among these, it is preferable that the starting material does not contain a solvent. In a case where no solvent is used, a reaction rate of the cyclic carboxylic acid compound in the heating step S02 described below can be increased. In addition, an amount of heat required for the heat treatment in the heating step S02 can be reduced. Therefore, production efficiency can be particularly improved. Note that "not containing a solvent" refers to a state in which a content of a solvent is 1% by mass or less of a total starting material.

On the other hand, in a case where the starting material contains a solvent, the fluidity of the starting material can be increased, and thus the handleability of the starting material can be favorable. However, in a case where an amount of the solvent is too large, an amount of heat required in the heating step S02 increases.

Therefore, in a case where the starting material contains a solvent, a content of the solvent is more than 1% by mass of the entire starting material, and a mass ratio of the solvent to the cyclic carboxylic acid compound is preferably 150 or less, and more preferably 15 or less.

Examples of the solvent include water, alcohol, alkylene glycol, aromatic hydrocarbons, ethers, and the like, and one alone or a mixture of two or more of these, or a mixture of at least one of these and other solvents is used.

Examples of the alcohol include methanol, ethanol, propanol, isopropyl alcohol, butanol, pentanol, hexanol, and the like.

Examples of the alkylene glycol include ethylene glycol, diethylene glycol, triethylene glycol, 1,2-propylene glycol, 1,3-propanediol, 1,4-butanediol, 1,5-pentanediol, 1,6-hexanediol, and the like.

Examples of aromatic hydrocarbons include benzene, toluene, xylene, phenol, cumene, mesitylene, naphthalene, tetraline, NMP (N-methyl-2-pyrrolidone), and the like.

Examples of the ethers include diphenyl ether, dimethyl ether, and the like.

### 2. Heating step S02

In the heating step S02, the starting material is subjected to a heat treatment. With this, a decarboxylation reaction is caused in the cyclic carboxylic acid compound. As a result, a cyclic compound is obtained.

Note that the decarboxylation reaction of the cyclic carboxylic acid compound is as shown in the above-described first embodiment.

Specifically, the present step is carried out as follows.

First, a starting material containing a cyclic carboxylic acid compound is placed in a reactor. The reactor is not particularly limited as long as the reactor is made of a material that does not cause denaturation of the starting material. In addition, although the reactor has a function of adjusting temperature, it may also have a function of adjusting pressure.

In addition, a catalyst may be placed in the reactor depending on the necessity. Examples of the catalyst include acid, alkali, hydrogen, transition metal compound, and the like. Among these, acid is preferably used. Acid is relatively easy to obtain and can take various forms, and thus tend to improve catalyst efficiency.

Examples of the acid include solid acid, inorganic acid, and organic acid. Note that in the following explanation, inorganic acid and organic acid may be referred to as "non-solid acids".

Among these, examples of the solid acid include zeolite, clay mineral, cation exchange resin, activated carbon, metal oxide, metal sulfide, metal salt, composite oxide, heteropolyacid, hydrated oxide, oxygen acid-supported metal oxide, and the like, and one or a combination of two or more of these may be used.

In addition, examples of the inorganic acid include sulfuric acid, carbonic acid, phosphoric acid, nitric acid, hydrochloric acid, and the like, and one or a mixture of two or more of these is used.

In addition, examples of the organic acid include various carboxylic acids and derivatives thereof such as aliphatic carboxylic acids (fatty acids), various sulfonic acids and derivatives thereof, and the like, and one or a mixture of two or more of these is used. In addition, various carboxylic acids include monocarboxylic acids, dicarboxylic acids, and tricarboxylic acids. Various sulfonic acids include monosulfonic acids, disulfonic acids, and trisulfonic acids. Examples of derivatives include esters, amides, acid anhydrides, acid chlorides, and the like.

Examples of aliphatic carboxylic acids include saturated aliphatic monocarboxylic acids such as formic acid, acetic acid, propionic acid, butyric acid, isobutyric acid, valeric acid, isovaleric acid, pivalic acid, caproic acid, glycolic acid, lactic acid, gluconic acid, and pyruvic acid, unsaturated aliphatic monocarboxylic acids such as acrylic acid, methacrylic acid, oleic acid, linoleic acid, and α-linolenic acid, saturated aliphatic dicarboxylic acids such as oxalic acid, malonic acid, succinic acid, malic acid, tartaric acid, α-ketoglutaric acid, glutaric acid, 2-oxoglutaric acid, and adipic acid, unsaturated aliphatic dicarboxylic acids such as maleic acid and fumaric acid, and tricarboxylic acids such as citric acid, isocitric acid, and aconitic acid.

In a case where a solid acid is used as the acid, the addition amount of the solid acid to the starting material is not particularly limited, but is preferably an amount such that the amount of the solid acid is 0.01 to 1.5 mol per 1.0 mol, and more preferably an amount such that the amount of the solid acid is 0.10 to 1.0 mol per 1.0 mol of the cyclic carboxylic acid compound. With this, a necessary and sufficient amount of catalyst acts on the cyclic carboxylic acid compound. As a result, it is possible to sufficiently increase the yield of the cyclic compound, and to suppress the negative effect due to an excessive amount of the catalyst, for example, a defect such as a decrease in the separability of the cyclic compound.

In addition, in a case where a non-solid acid is used as the acid, the addition amount of the non-solid acid to the starting material is not particularly limited, but is preferably an amount such that the addition concentration of the non-solid acid is 0.1 to 10.0 [mol%], more preferably an amount such that the addition concentration of the non-solid acid is 0.4 to 5.0 [mol%], and further more preferably an amount such that the addition concentration of the non-solid acid is 0.7 to 3.0 [mol%] to the total of the cyclic carboxylic acid compound and the non-solid acid. With this, a necessary and sufficient amount of catalyst acts on the cyclic carboxylic acid compound. As a result, it is possible to sufficiently increase the yield of the cyclic compound and to suppress the negative effect due to an excessive amount of the catalyst, for example, a defect such as a decrease in the separability of the cyclic compound.

In addition, in a case where a non-solid acid is used as the acid, the melting point of the non-solid acid is preferably 350°C or lower, and more preferably 300°C or lower. With this, the non-solid acid becomes a liquid or gas during the heat treatment, and thus the reaction system between the starting material and the non-solid acid becomes more homogeneous. As a result, the decarboxylation reaction can be particularly promoted.

In addition, in a case where a non-solid acid is used as the acid, the boiling point of the non-solid acid is preferably 80°C or higher, more preferably 90°C or higher and 350°C or lower, and further more preferably 100°C or higher and 250°C or lower. With this, the probability that the non-solid acid becomes a gas during the heat treatment is increased. As a result, non-solid acid can act particularly like a homogeneous catalyst, and particularly promote decarboxylation reactions.

Note that in a case where the cyclic compound to be produced is purified and recovered by evaporation separation, the boiling point of the non-solid acid is preferably higher than the boiling point of the cyclic compound to be produced. Specifically, the difference obtained by subtracting the boiling point of the cyclic compound from the boiling point of the non-solid acid is preferably higher than 0°C, more preferably 10°C to 250°C, and further more preferably 30°C to 200°C. In a case where the boiling point difference is within the above range, the separability of the cyclic compound in evaporation separation can be further improved. In other words, when the cyclic compound is evaporated and recovered as the temperature rises, it is possible to suppress the non-solid acid from evaporating together.

Note that even in a case where the boiling point difference is outside the above range, the cyclic compound can be purified and recovered by adding another separation treatment.

In addition, the catalyst may be placed in the reactor first, or the starting material and the catalyst may be placed simultaneously in the reactor. Moreover, the starting material may contain a catalyst together with the cyclic carboxylic acid compound.

Next, a heat treatment is carried out to heat the inside of the reactor. With this, the decarboxylation reaction is caused in the cyclic carboxylic acid compound contained in the starting material, and strong carbon-carbon bonds are broken. As a result, a cyclic compound is obtained.

The temperature in the heat treatment varies slightly depending on the presence or absence of a catalyst, but is preferably 150°C to 350°C, more preferably 180°C to 300°C, and further more preferably 200°C to 290°C. By carrying out the heat treatment at this temperature, the decarboxylation reaction can be further promoted. With this, the cyclic compound can be efficiently produced at a low cost.

In addition, in a case where the heating temperature is less than the lower limit value, the efficiency of the decarboxylation reaction decreases, and there is a concern that the yield of the cyclic compound decreases. On the other hand, in a case where the heating temperature exceeds an upper limit value, the yield will hardly increase, but energy consumption increases, leading to an increase in production cost.

The heating temperature may be adjusted depending on a melting point of the cyclic carboxylic acid compound. The heating temperature is preferably higher than the melting point of the cyclic carboxylic acid compound, more preferably 10°C or higher than the melting point. With this, since the reaction system becomes more homogeneous, the decarboxylation reaction becomes more marked. As a result, the yield of the cyclic compound can be further increased.

For example, since the melting point of 4-hydroxybenzoic acid is 214°C, the heating temperature in a case where the starting material containing 4-hydroxybenzoic acid is subjected to a heat treatment is preferably higher than 214°C and 350°C or less, and more preferably 224°C or higher and 290°C or lower.

Furthermore, since the melting point of 3,4-dihydroxybenzoic acid is 221°C, the heating temperature in a case where the starting material containing 3,4-dihydroxybenzoic acid is subjected to a heat treatment is preferably higher than 221°C and 350°C or lower, and more preferably 231°C or higher and 290°C or lower.

On the other hand, since the melting point of 4-aminobenzoic acid is 187°C, the heating temperature in a case where the starting material containing 4-aminobenzoic acid is subjected to a heat treatment is preferably higher than 187°C and 350°C or lower, and more preferably 197°C or higher and 290°C or lower.

In addition, a heating time at such a heating temperature is appropriately set in consideration of the reaction rate of the decarboxylation reaction and other reaction conditions, but as an example, the heating time is preferably 5 minutes or more and 24 hours or less, more preferably 10 minutes or more and 12 hours or less, and further more preferably 30 minutes or more and 6 hours or less.

A pressure inside the reactor during the heat treatment is not particularly limited, and may be normal pressure, less than normal pressure, or above normal pressure. Normal pressure refers to 86 kPa or more and 106 kPa or less. In a case where the pressure is normal pressure, the pressure resistance of the reactor can be lowered, and thus the reactor can be easily enlarged.

On the other hand, in a case where the pressure inside the reactor is lower than normal pressure, the volatility of the cyclic compound obtained after the decarboxylation reaction can be increased. With this, the cyclic compound can be purified by condensing the volatilized cyclic compound, and a cyclic compound with higher purity can be produced.

The specific pressure is preferably 0.01 atm (1 kPa) or more and less than normal pressure, and more preferably 0.1 atm or more and less than 0.7 atm (71 kPa). In addition, in a case where the pressure in the heat treatment is less than the lower limit value, there is a concern that the volatility of the cyclic carboxylic acid compound contained in the starting material also increases, and the yield decreases.

On the other hand, in a case where the pressure inside the reactor is set above normal pressure, the temperature inside the reactor can be raised to a temperature higher than a boiling point of the solvent. With this, the decarboxylation reaction becomes more marked. As a result, the yield of the cyclic compound can be further increased.

The specific pressure is preferably greater than normal pressure and 40 MPa or less, and more preferably 200 kPa or more and 10 MPa or less. This eliminates the need to increase the pressure resistance of the reactor more than necessary, making it easier to reduce costs.

In addition, the atmosphere inside the reactor is not particularly limited, and may be, for example, an air atmosphere, an inert gas atmosphere, or the like. Among these, an inert gas atmosphere may be selected when suppressing side reactions such as oxidation of a cyclic compound. Examples of the inert gas include nitrogen, carbon dioxide, argon, and the like.

The reactor may be a batch reactor, a semi-batch reactor, a continuous reactor, or a flow reaction system reactor.

Moreover, since the cyclic carboxylic acid compound becomes a gas under heating conditions of the boiling point or higher of the cyclic carboxylic acid compound, the reaction mode is preferably a fixed bed type, a moving bed type, or a fluidized bed type. Among these, the fixed bed type is particularly preferable because the reactor structure is not complicated and the construction cost is relatively low. In the case of heating, a tube heating furnace type that heats from the outside can be used.

Furthermore, the reactor may have a structure that can seal the starting material, or may have a structure that does not seal the starting material. In addition, alternatively, refluxing operation may be carried out in the reactor. With this, the yield and purity of the cyclic compound can be increased.

In addition, after the cyclic compound is produced, a step of separating or purifying the cyclic compound may be provided depending on the necessity.

### (Cyclic compound)

The cyclic compound according to the present embodiment is obtained by the production method described above.

The cyclic compound according to the present embodiment is a cyclic compound produced by causing a decarboxylation reaction in the cyclic carboxylic acid compound by subjecting a starting material containing the cyclic carboxylic acid compound derived from biomass to a heat treatment. The concentration of radioactive carbon ¹⁴C in all carbon atoms of this cyclic compound is 80% to 100%, when the concentration of radioactive carbon ¹⁴C in circulating carbon as of 1950 is taken as 100%. In this cyclic compound, a proportion of the main component having a single molecular structure is 90 to 100 mol%.

Such a cyclic compound contains a main component having a single molecular structure with high purity, and thus the quality becomes stable. Therefore, the cyclic compound suitable for various uses is obtained. Furthermore, since the concentration of radioactive carbon ¹⁴C in all carbon atoms of the cyclic compound is sufficiently high, a cyclic compound that greatly contributes to carbon neutrality is obtained. Note that the quality includes, for example, various physical properties such as melting point, boiling point, solubility, appearance, and coloration.

In addition, the concentration of radioactive carbon ¹⁴C in all carbon atoms of a cyclic compound is obtained by measuring a content of radioactive carbon ¹⁴C in a sample based on the biobased concentration test standard ASTM D6866-20 established by the American Society for Testing and Materials (ASTM), and calculating from the measurement result and the concentration of radioactive carbon ¹⁴C in circulating carbon as of 1950, for example. Note that this test standard specifies a plurality of analysis methods, and among these, analysis method B (AMS method) is preferably used. For analysis method B, for example, an analyzer such as an accelerator mass spectrometer Pelletron AMS manufactured by NEC Corporation is used.

The proportion of main components having a single molecular structure refers to a molar fraction of molecules (main components) having a single structure in a cyclic compound when molecules with different structures are included in the cyclic compound. The proportion of the main components is measured, for example, by gas chromatography mass spectrometry (GC/MS).

In addition, the above-described main components are preferably aromatic compounds derived from biomass. The aromatic compound derived from biomass refers to an aromatic compound in which all of the contained carbon atoms are radioactive carbon ¹⁴C. The cyclic compound containing such compounds as main components is particularly useful in various applications such as chemical products, fragrances, cosmetics, pharmaceuticals, and agricultural chemicals.

Specific examples of aromatic compounds derived from biomass include biomass-derived phenols, biomass-derived aromatic amines, biomass-derived aromatic aldehydes, biomass-derived aromatic alcohols, or derivatives thereof.

Examples of biomass-derived phenols include, in addition to phenol (hydroxybenzene), catechol, resorcinol, benzene diols (dihydroxybenzene) such as hydroquinone, benzene triol (trihydroxybenzene) such as hydroxynol, phloroglucinol, and pyroganol), o-cresol, m-cresol, p-cresol, 2,4-dimethylphenol, 2,6-dimethylphenol, 2,3,6-trimethylphenol, 2,4,6-trimethylphenol, 5-isopropyl-2-methylphenol, 4-isopropylphenol, 4-cyclohexylphenol, 4-phenylphenol, 3-pentadecylphenol, 3-pentadecylphenol monoene, 3-pentadecylphenol diene, 3-pentadecylphenol triene, catechol monoalkyl ether, hydroquinone monoalkyl ether, methyl 4-hydroxyphenylacetate, 1-naphthol, 2-naphthol, bisphenol A, 2,6-dimethoxyphenol, lignin, cashew oil, cardanol, cardol, 2-methylcardol, anacardic acid, and the like.

Examples of biomass-derived aromatic amines include aniline, o-toluidine, p-toluidine, o-anisidine, p-anisidine, 2,6-dimethylaniline, 2,4,6-trimethylaniline, 2,6-diethylaniline, 4-fluoroaniline, 4-chloroaniline, 2-aminophenol, 3-aminophenol, 4-aminophenol, N-methylaniline, and the like.

Examples of biomass-derived aromatic aldehydes include p-anisaldehyde, 3,4-dimethoxybenzaldehyde, 2,4-dimethoxybenzaldehyde, 2,5-dimethoxybenzaldehyde, 2,4,6-trimethoxybenzaldehyde, 4-ethoxybenzaldehyde, 4-butoxybenzaldehyde, 4-t-butoxybenzaldehyde, 4-cyclohexyloxybenzaldehyde, 3,4-methylenedioxybenzaldehyde, 4-(N,N-dimethylamino)benzaldehyde, 4-(N,N-diethylamino) benzaldehyde, 4-(N-methyl-N-phenylamino)benzaldehyde, 2-methoxynaphthalene-1-carbaldehyde, anthracene-9-carbaldehyde, 1-methoxyanthracene-9-carbaldehyde, naphthacene-5-carbaldehyde, and the like.

Examples of biomass-derived aromatic alcohols include benzyl alcohol (phenylmethanol), β-phenylethyl alcohol (phenylethanol), hydrocinnamyl alcohol (phenylpropanol), phenylbutanol, phenylpentanol, phenylhexanol, phenyloctanol, phenyldecanol, salicyl alcohol, gentisin alcohol, protocatequil alcohol, cuminyl alcohol, α-cumyl alcohol, cinnamyl alcohol, benzhydryl alcohol, trityl alcohol, hydrobenzoin, benzopinacol, phthalyl alcohol, isophthalyl alcohol, terephthalyl alcohol, and the like.

In addition, examples of the derivative include salts, hydrates, solvates, and the like.

In addition, the cyclic compound may contain non-biomass-derived compounds or other components as impurities, in addition to the main component.

In addition, the method for producing a cyclic compound according to the present embodiment includes a heating step S02. In the heating step S02, the starting material containing the cyclic carboxylic acid compound derived from biomass is subjected to a heat treatment. With this, a decarboxylation reaction is caused in the cyclic carboxylic acid compound to obtain a cyclic compound containing radioactive carbon ¹⁴C. The concentration of radioactive carbon ¹⁴C in all carbon atoms of this cyclic compound is 80% to 100%, when the concentration of radioactive carbon ¹⁴C in circulating carbon as of 1950 is taken as 100%. In this cyclic compound, a proportion of the main component having a single molecular structure is 90 to 100 mol%.

According to such a production method, it is possible to produce a cyclic compound having a high proportion of the main component having a single molecular structure as described above and a high concentration of radioactive carbon ¹⁴C. That is, according to the method for producing a cyclic compound according to the present embodiment, it is possible to produce a cyclic compound that greatly contributes to carbon neutrality and has stable quality.

Hereinabove, although the cyclic compound and the method for producing a cyclic compound of the present invention are described based on the embodiments, the present invention is not limited thereto.

For example, in the method for producing a cyclic compound of the present invention, an optional step may be added to each of the embodiments.

### [Examples]

Next, specific examples of the present invention will be described.

### 3. Production of cyclic compound (phenol)

### 3. 1. Example 1A

First, a starting material formed of biomass-derived 4-hydroxybenzoic acid was prepared as a cyclic carboxylic acid compound. This starting material was solvent-free and solid at room temperature.

Next, 0.05 g of the starting material and 0.05 g of a catalyst were charged into a closed reactor, and a heat treatment was carried out at 230°C for 30 minutes. With this, a reactant containing phenol, which is a cyclic compound, was obtained. Note that zeolite having the characteristics shown in Table 1 was used as the catalyst. "-" in Table 1 indicates that there is no data.

### 3. 2. Examples 2A to 15A

A reactant containing phenol, which is a cyclic compound, was obtained in the same manner as in Example 1A, except that the catalyst was changed to a catalyst having the characteristics shown in Table 1.

### 3. 3. Example 16A

A reactant containing phenol, which is a cyclic compound, was obtained in the same manner as in Example 4A, except that 0.3 g of solvent (diphenyl ether) was added to the starting material.

### 3. 4. Example 17A

A reactant containing phenol, which is a cyclic compound, was obtained in the same manner as in Example 9A, except that 0.5 g of solvent (diphenyl ether) was added to the starting material.

### 3. 5. Comparative Examples 1A to 4A

A reactant containing phenol, which is a cyclic compound, was obtained in the same manner as in Example 1A, except that the catalyst was changed to a catalyst having the characteristics shown in Table 1.

### 3. 6. Comparative Example 5A

An attempt was made to obtain a reactant containing phenol, which is a cyclic compound, in the same manner as in Example 1A, except that no catalyst was used.

### 4. Evaluation of cyclic compound (phenol)

### 4. 1. Yield of cyclic compound

The reactant obtained in 3. was analyzed using a highspeed liquid chromatograph ("LaChrom Elite" manufactured by Hitachi High-Technologies Corporation, analytical column: "Inertsil ODS-4" manufactured by GL Sciences, Inc.). Next, a molar amount of the charged cyclic carboxylic acid compound and a molar amount of the produced cyclic compound were calculated. Then, a yield of the cyclic compound was calculated based on the following formula.

Yield of cyclic compound (%) = molar amount of produced cyclic compound/molar amount of charged cyclic carboxylic acid compound × 100

The calculation results are shown in Table 1.

### 4. 2. Coloring of cyclic compound

The degree of coloring of the cyclic compound was observed by visually observing the coloring of the reactant obtained in 3. The observation results were then evaluated in accordance with the following evaluation criteria.
A: No coloring
B: Less coloring
C: Slightly more coloring (more than B, less than D)
D: A lot of coloring in some parts
E: A lot of coloring throughout the compound

The following evaluation results are shown in Table 1.

### 4. 3. Comprehensive evaluation

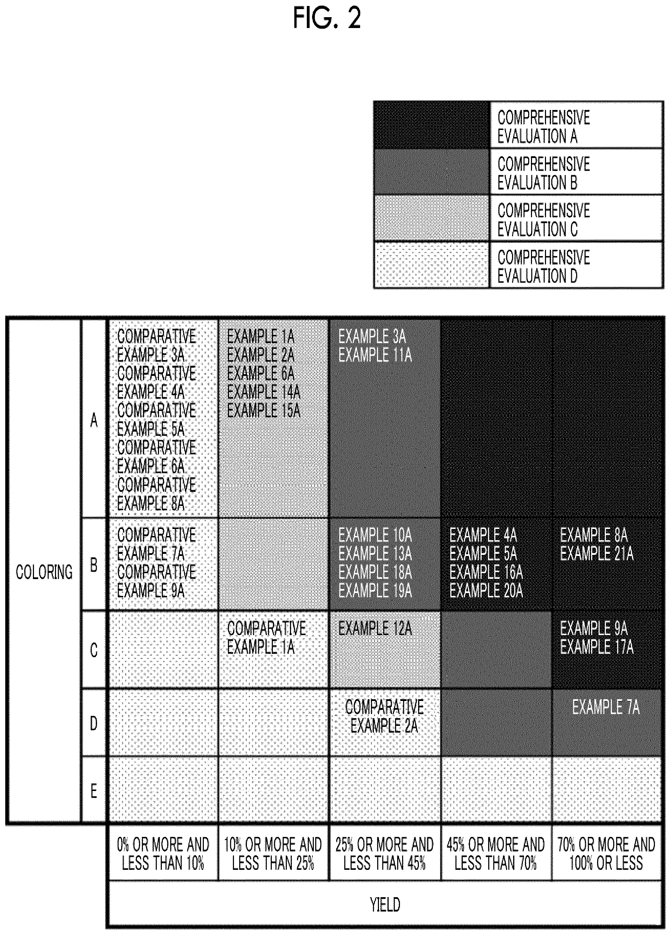

Comprehensive evaluation was carried out on the yield and coloring described above in accordance with the evaluation criteria shown in FIG. 2. The evaluation results are shown in Table 1.

FIG. 2 is a diagram showing evaluation criteria for comprehensive evaluation of the yield and the degree of coloring of the cyclic compound produced in each example and each comparative example. The evaluation criteria shown in FIG. 2 are divided into four stages based on both the yield and the degree of coloring of the cyclic compound. According to this evaluation criteria, A has the highest comprehensive evaluation, followed by B, and then the lowest D. By applying the yield and the degree of coloring of the cyclic compound produced in each Example and each Comparative Example to the evaluation criteria shown in FIG. 2, a comprehensive evaluation of the production of the cyclic compound in each example and each comparative example was carried out.

### [Table 1]

**Table 1**

| | Production conditions of cyclic compound | | | | | | | | | | Evaluation results of cyclic compound | | |
|---|---|---|---|---|---|---|---|---|---|---|---|---|---|
| | Starting material | | Catalyst | | | | | | | Heating temperature | | | |
| | Cyclic carboxylic acid compound | Solvent | Type | SiO₂/Al₂O₃ ratio | Acid amount by NH₃-TPD | Cation | Pore diameter | Crystal form of zeolite | Structural code including crystal form | | Yield | Coloring | Comprehensive evaluation |
| | - | - | - | - | mmol/g | - | nm | - | - | °C | % | - | - |
| Example 1A | | | | 18 | 2.6 | NH4* | 0.48 | Ferrierite type | FER | | 12 | A | C |
| Example 2A | | | | 18 | - | K⁺ | | | | | 14 | A | C |
| Example 3A | | | | 23 | - | NH4* | 0.58 | ZSM-5 type | MFI | | 26 | A | B |
| Example 4A | | | | 24 | 1.8 | H⁺ | | | | | 65 | B | A |
| Example 5A | | | | 40 | 1.3 | H⁺ | | | | | 46 | B | A |
| Example 6A | | | | 18 | - | Organic | 0.65 | Beta type | BEA | | 21 | A | C |
| Example 7A | | | | 28 | 1.2 | H⁺ | | | | | 81 | D | B |
| Example 8A | | | | 40 | 0.5 | H⁺ | | | | | 74 | B | A |
| Example 9A | | | | 40 | 0.8 | H⁺ | | | | | 87 | C | A |
| Example 10A | | None | | 500 | - | H⁺ | | | | | 44 | B | B |
| Example 11A | 4-hydroxybenzoic acid | | Zeolite | 18 | - | Na⁺ | 0.7 | Mordenite type | MOR | 230 | 26 | A | B |
| Example 12A | | | | 15 | 2.0 | H⁺ | | | | | 26 | C | C |
| Example 13A | | | | 18 | 0.7 | H⁺ | | | | | 40 | B | B |
| Comparative Example A | | | | 6.1 | - | K⁺ | 0.8 | L type | LTL | | 15 | C | D |
| Example 14A | | | | 25 | - | NH4* | 0.9 | Y type | FAU | | 15 | A | C |
| Comparative Example 2A | | | | 5.5 | - | Na⁺ | | | | | 43 | D | D |
| Comparative Example 3A | | | | 5.5 | 0.7 | H⁺ | | | | | 9 | A | D |
| Example 15A | | | | 14 . 9 | 0.1 | H⁺ | | | | | 17 | A | C |
| Comparative Example 4A | | | | 600 | < 0.1 | H⁺ | | | | | 5 | A | D |
| Example 16A | | Present | | 24 | 1.8 | H⁺ | 0.58 | ZSM-5 type | MFI | | 55 | B | A |
| Example 17A | | | | 40 | 0.8 | H⁺ | 0.65 | Beta type | BEA | | 75 | C | A |
| Comparative Example 5A | | None | No catalyst | | | | | | | | 9 | A | D |

As is clear from Table 1 and FIG. 2, in each example, the comprehensive evaluation was higher than in each comparative example, and thus it was possible to produce a cyclic compound with a high yield and less coloring. In each example, the SiO₂/Al₂O₃ ratio of the zeolite contained in the catalyst falls within a predetermined range. From this, it became clear that a high-quality cyclic compound can be produced in a high yield by using a zeolite having an SiO₂/Al₂O₃ ratio within a predetermined range as a catalyst.

In addition, in a case where an acid amount of the zeolite determined by the NH₃-TPD method is within a predetermined range, in a case where the zeolite contains hydrogen, it was recognized that any of the yield or the coloring becomes particularly favorable in a case where a skeleton structure of the zeolite has a predetermined crystal form and the like.

Although not shown in Table 1, in a case where the same test as described above was carried out by changing a temperature of the heat treatment to 180°C, 200°C, and 250°C, evaluation results showing the same tendency as described above were obtained.

### 5. Production of cyclic compound (catechol or aniline)

### 5. 1. Examples 18A and 19A

### 5. 2. Comparative Examples 6A and 7A

A reactant containing catechol, which is a cyclic compound, was obtained in the same manner as in Comparative Example 1A, except that the cyclic carboxylic acid compound was changed to 3,4-dihydroxybenzoic acid.

### 5. 3. Examples 20A and 21A

A reactant containing aniline, which is a cyclic compound, was obtained in the same manner as in Example 1A, except that the cyclic carboxylic acid compound was changed to 4-aminobenzoic acid.

### 5. 4. Comparative Examples 8A and 9A

A reactant containing aniline, which is a cyclic compound, was obtained in the same manner as in Comparative Example 1A, except that the cyclic carboxylic acid compound was changed to 4-aminobenzoic acid.

### 6. Evaluation of cyclic compound (catechol or aniline)

### 6. 1. Yield of cyclic compound

For the reactant obtained in 5., the yield of the cyclic compound was calculated in the same manner as in 4. The calculation results are shown in Tables 2 and 3.

### 6. 2. Coloring of cyclic compound

For the reactant obtained in 5., the coloring of the cyclic compound was evaluated in the same manner as in 4. The evaluation results are shown in Tables 2 and 3.

### 6. 3. Comprehensive evaluation

For the above-described yield and coloring, comprehensive evaluation was carried out in the same manner as in 4. The evaluation results are shown in Tables 2 and 3.

### [Table 2]

**Table 2**

| | Production conditions of cyclic compound | | | | | | | | | | Evaluation results of cyclic compound | | |
|---|---|---|---|---|---|---|---|---|---|---|---|---|---|
| | Starting material | | Catalyst | | | | | | | Heating temperature | | | |
| | Cyclic carboxylic acid compound | Solvent | Type | SiO₂/Al₂O₃ ratio | Acid amount by NH₃-TPD | Cation | Pore diameter | Crystal form of zeolite | Structural code including crystal form | | Yield | Coloring | Comprehensive evaluation |
| | - | - | - | - | mmol/g | - | nm | - | - | °C | % | - | - |
| Example 18A | 3,4-dihydroxybenzoic acid | None | Zeolite | 24 | 1.8 | H⁺ | 0.58 | ZSM-5 type | MFI | 190 | 30 | B | B |
| Example 19A | | | | 40 | 0.8 | H⁺ | 0.65 | Beta type | BEA | | 40 | B | B |
| Comparative Example 6A | | | | 5.5 | 0.7 | H⁺ | 0.9 | Y type | FAU | | 8 | A | D |
| Comparative Example 7A | | | | 600 | < 0.1 | H⁺ | | | | | 4 | B | D |

### [Table 3]

**Table 3**

| | Production conditions of cyclic compound | | | | | | | | | | Evaluation results of cyclic compound | | |
|---|---|---|---|---|---|---|---|---|---|---|---|---|---|
| | Starting material | | Catalyst | | | | | | | Heating temperature | | | |
| | Cyclic carboxylic acid compound | Solvent | Type | SiO_{2/}Al₂O₃ ratio | Acid amount by NH₃-TPD | Cation | Pore diameter | Crystal form of zeolite | Structural code including crystal form | | Yield | Coloring | Comprehensive evaluation |
| | - | - | - | - | mmol/g | - | nm | - | - | °C | % | - | - |
| Example 20A | 4-aminobenzoic acid | None | Zeol ite | 24 | 1.8 | H⁺ | 0.58 | ZSM-5 type | MFI | 220 | 55 | B | A |
| Example 21A | | | | 40 | 0.8 | H⁺ | 0.65 | Beta type | BEA | | 75 | B | A |
| Comparative Example 8A | | | | 5.5 | 0.7 | H⁺ | 0.9 | Y type | FAU | | 7 | A | D |
| Comparative Example 9A | | | | 600 | < 0.1 | H⁺ | | | | | 3 | B | D |

As is clear from Tables 2 and 3, even in a case where the cyclic carboxylic acid compound contained in the starting material was changed, in each example, a high-quality cyclic compound was produced in a higher yield than in each comparative example.

### 7. Production of cyclic compound (phenol) using non-solid acid

### 7. 1. Example 1B

First, a starting material containing 4-hydroxybenzoic acid derived from biomass was prepared as a cyclic carboxylic acid compound. This starting material was solvent-free and solid at room temperature.

Next, 0.05 g of the starting material and the non-solid acid were charged into a closed reactor, an initial pressure in the reactor was set to normal pressure (100 kPa), and a heat treatment was carried out at 230°C for 30 minutes. With this, a reactant containing phenol, which is a cyclic compound, was obtained. Note that the non-solid acids shown in Table 4 were used.

### 7. 2. Examples 2B to 12B

A reactant containing phenol, which is a cyclic compound, was obtained in the same manner as in Example 1B, except that the non-solid acid was changed to a non-solid acid having the characteristics shown in Table 4.

### 7. 3. Example 13B

A reactant containing phenol, which is a cyclic compound, was obtained in the same manner as in Example 7B, except that 0.3 g of solvent (diphenyl ether) was added to the starting material.

### 7. 4. Example 14B

A reactant containing phenol, which is a cyclic compound, was obtained in the same manner as in Example 8B, except that 0.5 g of solvent (diphenyl ether) was added to the starting material.

### 7. 5. Comparative Example 1B

A reactant containing phenol, which is a cyclic compound, was obtained in the same manner as in Example 1B, except that a non-solid acid was not used.

### 8. Evaluation of cyclic compound (phenol) produced using non-solid acid

### 8.1. Yield of cyclic compound

The reactant obtained in 7. was analyzed using a highspeed liquid chromatograph ("LaChrom Elite" manufactured by Hitachi High-Technologies Corporation, analytical column: "Inertsil ODS-4" manufactured by GL Sciences, Inc.). Next, a molar amount of the charged cyclic carboxylic acid compound and a molar amount of the produced cyclic compound were calculated. Then, a yield of the cyclic compound was calculated based on the following formula. Yield of cyclic compound (%) = molar amount of produced cyclic compound/molar amount of charged cyclic carboxylic acid compound × 100

The calculation results are shown in Table 4.

### 8. 2. Separability of cyclic compound

For the reactant obtained in 7., the purity of the cyclic compound (phenol) was measured. Then, the separability of the cyclic compound was evaluated by comparing the measurement results with the following evaluation criteria.

A: The purity of the cyclic compound is particularly high.
B: The purity of the cyclic compound is slightly high.
C: The purity of the cyclic compound is slightly low.
D: The purity of the cyclic compound is particularly low.

The above evaluation results are shown in Table 4.

### 8. 3. Coloring of cyclic compound

The degree of coloring of the cyclic compound was observed by visually observing the coloring of the reactant obtained in 7. The observation results were then evaluated in accordance with the following evaluation criteria.

A: No coloring
B: Less coloring
C: Slightly more coloring (more than B, less than D)
D: A lot of coloring in some parts
E: A lot of coloring throughout the compound

The above evaluation results are shown in Table 4.

### 8. 4. Comprehensive evaluation

Comprehensive evaluation of the above-described yield, separability, and coloring was carried out in accordance with the following evaluation criteria. The evaluation results are shown in Table 4.

A: The yield is higher than Comparative Example 1B (difference is 2% or more), and both of separability and coloring are rated B or higher
B: The yield is higher than Comparative Example 1B (difference is 2% or more), and one of separability or coloring is rated C, and the other is rated B or higher
C: The yield is higher than Comparative Example 1B (difference is less than 2%), and both of separability and coloring are rated C or higher
D: The yield is the same as or lower than Comparative Example 1B

The above evaluation results are shown in Table 4.

### [Table 4]

**Table 4**

| | Production conditions of cyclic compound | | | | | | | | | Evaluation results of cyclic compound | | | |
|---|---|---|---|---|---|---|---|---|---|---|---|---|---|
| | Starting material | | Non-solid acid | | | | | | Heating temperature | | | | |
| | Cyclic carboxylic acid compound | Solvent | Type | | Addition concentration | Acid dissociation constant pKa | Boiling point | Boiling point difference between cyclic compound | | Yield | Separability | Coloring | Comprehensive evaluation |
| | - | -- | - | - | mol% | - | °C | °C | °C | % | - | - | - |
| Example 1B | 4-hydroxybenzoic acid | None | Inorganic acid | Sulfuric acid | 0.5 | -3 | 337 | 156 | 230 | 10 | A | C | C |
| Example 2B | | | | | 1.0 | -3 | 337 | 156 | | 20 | A | C | B |
| Example 3B | | | | | 2.0 | -3 | 337 | 156 | | 40 | A | C | B |
| Example 4B | | | Organic acid | Formic acid | 0.5 | 3.75 | 101 | 80 | | 10 | B | A | C |
| Example 5B | | | | | 1.0 | 3.75 | 101 | 80 | | 12 | B | A | A |
| Example 6B | | | | | 2.0 | 3.75 | 101 | 80 | | 12 | B | A | A |
| Example 7B | | | | Succinic acid | 0.5 | 4.2 | 235 | 54 | | 10 | B | A | C |
| Example 8B | | | | | 1.0 | 4.2 | 235 | 54 | | 11 | B | A | A |
| Example 9B | | | | | 2.0 | 4.2 | 235 | 54 | | 11 | B | A | A |
| Example 10B | | | | Pivalic acid | 0.5 | 5.01 | 164 | 17 | | 10 | C | A | C |
| Example 11B | | | | | 1.0 | 5.01 | 164 | 17 | | 10 | C | A | C |
| Example 12B | | | | | 2.0 | 5.01 | 164 | 17 | | 10 | C | A | C |
| Example 13B | | Present | | Succinic acid | 0.5 | 4.2 | 235 | 54 | | 10 | B | A | C |
| Example 14B | | | | | 1.0 | 4.2 | 235 | 54 | | 11 | B | A | A |
| Comparative Example 1B | | None | No addition of non-solid acid | | | | | | | 2 | - | A | D |

As is clear from Table 4, in each Example, the cyclic compound was able to be produced in a higher yield than in Comparative Example 1B. Furthermore, in some of the examples, the separability or degree of coloring of the cyclic compound was favorable.

Note that although not shown in Table 4, in a case where the same test as described above was carried out by changing a temperature of the heat treatment to 180°C, 200°C, and 250°C, evaluation results showing the same tendency as described above were obtained.

### 9. Production of cyclic compound (catechol or aniline) using non-solid acid

### 9. 1. Examples 15B to 17B

A reactant containing catechol, which is a cyclic compound, was obtained in the same manner as in Examples 1B to 3B, except that the cyclic carboxylic acid compound was changed to 3,4-dihydroxybenzoic acid and the heating temperature was set to 190°C.

### 9. 2. Comparative Example 2B

A reactant containing catechol, which is a cyclic compound, was obtained in the same manner as in Comparative Example 1B, except that the cyclic carboxylic acid compound was changed to 3,4-dihydroxybenzoic acid and the heating temperature was set to 190°C.

### 9. 3. Examples 18B to 20B

A reactant containing aniline, which is a cyclic compound, was obtained in the same manner as in Examples 7B to 9B, except that the cyclic carboxylic acid compound was changed to 4-aminobenzoic acid.

### 9. 4. Comparative Example 3B

A reactant containing aniline, which is a cyclic compound, was obtained in the same manner as in Comparative Example 1B, except that the cyclic carboxylic acid compound was changed to 4-aminobenzoic acid.

10. Evaluation of cyclic compound (catechol or aniline) produced using non-solid acid

10. 1. Yield of cyclic compound

For the reactant obtained in 9., the yield of the cyclic compound was calculated in the same manner as in 8. The calculation results are shown in Tables 5 and 6.

### 10. 2. Separability of cyclic compound

For the reactant obtained in 9., the purity of the cyclic compound was measured, in the same manner as in 8., and the separability was evaluated based on the purity. The evaluation results are shown in Tables 5 and 6.

### 10. 3. Coloring of cyclic compound

For the reactant obtained in 9., coloring of the cyclic compound was evaluated in the same manner as in 8. The evaluation results are shown in Tables 5 and 6.

### 10. 4. Comprehensive evaluation

For the above-described yield, separability, and degree of coloring, comprehensive evaluation was carried out in the same manner as in 8. The evaluation results are shown in Tables 5 and 6. Note that as the evaluation criteria for the comprehensive evaluation, evaluation criteria in which Comparative Example 1B in the evaluation criteria used in 8. was replaced by Comparative Examples 2B and 3B were used.

### [Table 5]

**Table 5**

| | Production conditions of cyclic compound | | | | | | | | | Evaluation results of cyclic compound | | | |
|---|---|---|---|---|---|---|---|---|---|---|---|---|---|
| | Starting material | | Non-solid acid | | | | | | Heating temperature | | | | |
| | Cyclic carboxylic acid compound | Solvent | Type | | Addition concentration | Acid dissociation constant pKa | Boiling point | Boiling point difference between cyclic compound | | Yield | Separability | Coloring | Comprehensive evaluation |
| | - | - | - | - | mol% | - | °C | °C | °C | % | - | - | - |
| Example 15B | 3,4-dihydroxybenzoic acid | None | Inorganic acid | Sulfuric acid | 0.5 | 4.2 | 337 | 92 | 190 | 12 | B | C | B |
| Example 16B | | | | | 1.0 | 4.2 | 337 | 92 | | 13 | B | C | B |
| Example 17B | | | | | 2.0 | 4.2 | 337 | 92 | | 13 | B | C | B |
| Comparative Example 2B | | | No addition of non-solid acid | | | | | | | 8 | - | A | D |

### [Table 6]

**Table 6**

| | Production conditions of cyclic compound | | | | | | | | | Evaluation results of cyclic compound | | | |
|---|---|---|---|---|---|---|---|---|---|---|---|---|---|
| | Starting material | | Non-solid acid | | | | | | Heating temperature | | | | |
| | Cyclic carboxylic acid compound | Solvent | Type | | Addition concentration | Acid dissociation constant pKa | Boiling point | Boiling point difference between cyclic compound | | Yield | Separability | Coloring | Comprehensive evaluation |
| | - | - | - | - | mol% | - | °C | °C | °C | % | - | - | - |
| Example 18B | 4-aminobenzoic acid | None | Organic acid | Succinic acid | 0.5 | 4.2 | 235 | 51 | 240 | 11 | B | A | A |
| Example 19B | | | | | 1.0 | 4.2 | 235 | 51 | | 12 | B | A | A |
| Example 20B | | | | | 2.0 | 4.2 | 235 | 51 | | 13 | B | A | A |
| Comparative Example 3B | | | No addition of non-solid acid | | | | | | | 2 | - | A | D |

As is clear from Tables 5 and 6, even in a case where the cyclic carboxylic acid compound contained in the starting materials was changed, in each example, the cyclic compound was able to be produced in a higher yield than each comparative example.

### 11. Production of cyclic compound (phenol) using a combination of non-solid acid and solid acid

### 11. 1. Example 21B

0.05 g of the reactant obtained in Example 1B and 0.01 g of solid acid were charged into a closed reactor, an initial pressure in the reactor was set to normal pressure (100 kPa), and a heat treatment was carried out at 230°C for 15 minutes. With this, a reactant containing phenol, which is a cyclic compound, was obtained. Note that the solid acids shown in Table 7 were used.

### 11. 2. Examples 22B to 38B

A reactant containing phenol, which is a cyclic compound, was obtained in the same manner as in Example 21B, except that a solid acid having the characteristics shown in Table 7 was used.

### 11. 3. Example 39B

A reactant containing phenol, which is a cyclic compound, was obtained in the same manner as in Example 21B, except that 0.05 g of the reactant obtained in Example 7B was used. Note that the solid acids shown in Table 8 were used.

### 11. 4. Examples 40B to 56B

A reactant containing phenol, which is a cyclic compound, was obtained in the same manner as in Example 39B, except that the solid acid having the characteristics shown in Table 8 was used.

### 12. Evaluation of cyclic compound (phenol) produced using combination of non-solid acid and solid acid

For the reactant obtained in 11., the yield of the cyclic compound was calculated in the same manner as in 8. The calculation results are shown in Tables 7 and 8.

### [Table 7]

**Table 7**

| | Production conditions of cyclic compound | | | | | | | | | | Evaluation results of cyclic compound | |
|---|---|---|---|---|---|---|---|---|---|---|---|---|
| | Starting material | | Non-solid acid | Solid acid | | | | | | Heating temperature | | |
| | Cyclic carboxylic acid compound | Solvent | Type | Type | SiO_{2/}Al₂O₃ ratio | Acid amount by NH₃-TPD | Cation | Pore diameter | Crystal form of zeolite | | Yield | Increase range of yield |
| | - | - | - | - | - | mmol/g | - | nm | - | °C | % | % |
| Example 21B | | | | | 18 | 2.6 | NH4⁺ | 0.48 | Ferrierite type | | 11 | 1 |
| Example 22B | | | | | 18 | - | K⁺ | | | | 12 | 2 |
| Example 23B | | | | | 23 | - | NH4⁺ | 0.58 | ZSM-5 type | | 19 | 9 |
| Example 24B | | | | | 24 | 1.8 | H⁺ | | | | 41 | 31 |
| Example 25B | | | | | 40 | 1.3 | H⁺ | | | | 30 | 20 |
| Example 26B | | | | | 18 | - | Organic | 0.65 | Beta type | | 16 | 6 |
| Example 27B | | | | | 28 | 1.2 | H⁺ | | | | 50 | 40 |
| Example 28B | | | | | 40 | 0.5 | H⁺ | | | | 46 | 36 |
| Example 29B | 4-aminobenzoic acid | None | Sulfuric acid | Zeolite | 40 | 0.8 | H⁺ | | | 230 | 53 | 43 |
| Example 30B | | | | | 500 | - | H⁺ | | | | 29 | 19 |
| Example 31B | | | | | 18 | - | Na⁺ | 0.7 | Mordenite type | | 19 | 9 |
| Example 32B | | | | | 15 | 2.0 | H⁺ | | | | 19 | 9 |
| Example 33B | | | | | 18 | 0.7 | H⁺ | | | | 27 | 17 |
| Example 34B | | | | | 6.1 | - | K⁺ | 0.8 | L type | | 13 | 3 |
| Example 35B | | | | | 25 | - | NH4* | 0.9 | Y type | | 13 | 3 |
| Example 36B | | | | | 5.5 | 0.7 | H⁺ | | | | 11 | 1 |
| Example 37B | | | | | 14 . 9 | 0.1 | H⁺ | | | | 14 | 4 |
| Example 38B | | | | | 600 | < 0.1 | H⁺ | | | | 11 | 1 |

### [Table 8]

**Table 8**

| | Production conditions of cyclic compound | | | | | | | | | | Evaluation results of cyclic compound | |
|---|---|---|---|---|---|---|---|---|---|---|---|---|
| | Starting material | | Non-solid acid | Solid acid | | | | | | Heating temperature | | |
| | Cyclic carboxylic acid compound | Solvent | Type | Type | SiO_{2/}Al₂O₃ ratio | Acid amount by NH₃-TPD | Cation | Pore diameter | Crystal form of zeolite | | Yield | Increase range of yield |
| | - | - | - | - | - | mmol/g | - | nm | - | °C | % | % |
| Example 39B | | | | | 18 | 2.6 | NH4⁺ | 0.48 | Ferrierite type | | 11 | 1 |
| Example 40B | | | | | 18 | - | K⁺ | | | | 12 | 2 |
| Example 41B | | | | | 23 | - | NH4⁺ | 0.58 | ZSM-5 type | | 18 | 8 |
| Example 42B | | | | | 24 | 1.8 | H⁺ | | | | 39 | 29 |
| Example 43B | | | | | 40 | 1.3 | H⁺ | | | | 29 | 19 |
| Example 44B | | | | | 18 | - | Organic | 0.65 | Beta type | | 16 | 6 |
| Example 45B | | | | | 28 | 1.2 | H⁺ | | | | 48 | 38 |
| Example 46B | | | | | 40 | 0.5 | H⁺ | | | | 44 | 34 |
| Example 47B | 4-hydroxybenzoic acid | None | Succinic acid | Zeolite | 40 | 0.8 | H⁺ | | | 230 | 51 | 41 |
| Example 48B | | | | | 500 | - | H⁺ | | | | 28 | 18 |
| Example 49B | | | | | 18 | - | Na⁺ | 0.7 | Mordenite type | | 18 | 8 |
| Example 50B | | | | | 15 | 2.0 | H⁺ | | | | 18 | 8 |
| Example 51B | | | | | 18 | 0.7 | H⁺ | | | | 26 | 16 |
| Example 52B | | | | | 6.1 | - | K⁺ | 0.8 | L type | | 13 | 3 |
| Example 53B | | | | | 25 | - | NH4⁺ | 0.9 | Y type | | 13 | 3 |
| Example 54B | | | | | 5.5 | 0.7 | H⁺ | | | | 11 | 1 |
| Example 55B | | | | | 14 . 9 | 0.1 | H⁺ | | | | 14 | 4 |
| Example 56B | | | | | 600 | < 0.1 | H⁺ | | | | 11 | 1 |

As is clear from Tables 7 and 8, as a decarboxylation reaction was caused in the cyclic carboxylic acid compound in the presence of the non-solid acid, and the produced reactant was subjected to a heat treatment in the presence of a solid acid, a further increase in the yield was recognized. Specifically, in Examples 1B and 7B, the yield of phenol was 10%. As these were subjected to a heat treatment in the presence of a solid acid, a further increase in the yield was possible. From this result, it was confirmed that by using a non-solid acid and a solid acid in combination, the decarboxylation reaction can be particularly promoted and the yield can be further increased.

Note that the above example is an example in which a reactant produced in the presence of a non-solid acid (inorganic acid or organic acid) is further subjected to a heat treatment in the presence of a solid acid, but an experiment was carried out even in an example in which the non-solid acid and the solid acid were placed together in the reactor and subjected to a heat treatment. Although the experimental results are not shown in each table, the results showed the same tendency as Tables 7 and 8. Therefore, it was confirmed that in a case where a non-solid acid and a solid acid are used together, the non-solid acid and the solid acid may be used separately in terms of time or may be used overlappingly in terms of time.

### 13. Production of cyclic compound

### 13. 1. Example 1C

First, a starting material containing 4-hydroxybenzoic acid derived from biomass was prepared as a cyclic carboxylic acid compound. This starting material was solvent-free and solid at room temperature.

Next, the starting materials were charged into a closed reactor, a pressure inside the reactor was set to normal pressure (100 kPa), and a heat reflux treatment was carried out at 300°C for 1 hour.

Thereafter, the temperature inside the reactor was raised to 180°C, and the reflux treatment was stopped, the pressure was reduced to 20 kPa, and a cyclic compound containing phenol as a main component, which is an aromatic compound derived from biomass, was distilled and recovered.

### 13. 2. Example 2C

A cyclic compound having catechol as a main component, which is an aromatic compound derived from biomass, was distilled and recovered in the same manner as in Example 1C, except that the cyclic carboxylic acid compound was changed to 3,4-dihydroxybenzoic acid derived from biomass.

### 13. 3. Example 3C

A cyclic compound containing aniline as a main component, which is an aromatic compound derived from biomass, was distilled and recovered in the same manner as in Example 1C, except that the cyclic carboxylic acid compound was changed to 4-aminobenzoic acid derived from biomass.

### 13. 4. Comparative Example 1C

As a cyclic carboxylic acid compound, a commercially available reagent of 4-hydroxybenzoic acid derived from fossil resources was prepared. This reagent was solvent-free and solid at room temperature. A cyclic compound containing phenol as a main component, which is an aromatic compound derived from fossil resources, was distilled and recovered in the same manner as in Example 1C, except that this reagent was used as a starting material.

### 13. 5. Comparative Example 2C

A commercially available reagent of phenol as an aromatic compound derived from fossil resources was prepared, and this was used as the cyclic compound of Comparative Example 2.

### 13. 6. Comparative Example 3C

A commercially available reagent of lignin as an aromatic compound derived from biomass was prepared, and this was used as the cyclic compound of Comparative Example 3C. Note that lignin is a phenol containing the highest proportion of 2,6-dimethoxyphenol.

### 13. 7. Comparative Example 4C

A commercially available reagent of cashew oil was prepared as an aromatic compound derived from biomass, and this was used as the cyclic compound of Comparative Example 4C. Note that cashew oil is a phenol containing the highest proportion of 3-pentadecylphenoltriene.

Table 9 shows the attributes of the starting materials used in each example and each comparative example described above, and the attributes of the cyclic compounds produced from the starting materials.

### 14. Evaluation of cyclic compound

### 14. 1. Concentration of radioactive carbon ¹⁴C

For the recovered or prepared cyclic compound, a content of radioactive carbon ¹⁴C in all carbon atoms was measured based on the biobased concentration test standard ASTM D6866-20 established by the American Society for Testing and Materials (ASTM). In addition, from the measurement results and the concentration of radioactive carbon ¹⁴C in the circulating carbon as of 1950, the concentration of radioactive carbon ¹⁴C in all carbon atoms when the concentration of radioactive carbon ¹⁴C in the circulating carbon of the recovered or prepared cyclic compound as of 1950 was determined to be 100% was calculated. The calculation results are shown in Table 9.

### 14. 2. Proportion of main components

A proportion of main components of the recovered or prepared cyclic compound was measured using a total ion chromatogram obtained by pyrolysis gas chromatography mass spectrometry shown below. The measurement results are shown in Table 9.

### · Analyzer

Pyrolysis apparatus: PY-2020D, manufactured by Frontier Laboratories Ltd.
GC: HP6890, manufactured by Agilent Technologies
MS: HP5973, manufactured by Agilent Technologies

### · Analysis conditions

Pyrolysis temperature: 500°C
GC oven temperature: 40°C (held for 2 minutes) → 20°C/min → 320°C (held for 5 minutes)
MS interface temperature: 300°C
Column: Capillary column Ultra ALLOY-5 (length 30m, inner diameter 0.25 mm, film thickness 0.25 um)
Injection method: Split method (split ratio 50:1) Inlet temperature: 300°C
Carrier gas: He
Carrier gas flow rate: 1.2 mL/min
MS ionization method: EI (electron impact) method MS detection mass range: m/z = 29 to 550

### 14. 3. Contribution to carbon neutrality

A degree of contribution to carbon neutrality of the recovered or prepared cyclic compound was evaluated based on the following evaluation criteria.

A: Great contribution to carbon neutrality
B: Small contribution to carbon neutrality

The evaluation results are shown in Table 9.

### 14. 4. Quality stability

The quality stability of the recovered or prepared cyclic compound was evaluated in accordance with the following evaluation criteria.

A: Quality is stable
B: Quality is unstable

The evaluation results are shown in Table 9.

**[Table 9] Table 9**

| | | | Example 1C | Example 2C | Example 3C | Comparative Example 1C | Comparative Example 2C | Comparative Example 3C | Comparative Example 4C |
|---|---|---|---|---|---|---|---|---|---|
| Starting material | Cyclic carboxylic acid compound | - | 4-hydroxybenzoic acid | 3,4-dihydroxybenzoic acid | 4-aminobenzoic acid | 4-hydroxybenzoic acid | - | - | - |
| | Derived from starting material | - | Derived from biomass | Derived from biomass | Derived from biomass | Derived from fossil resources | - | - | - |
| Cyclic compound | Concentration of radioactive carbon ¹⁴C | % | 100 | 100 | 100 | 0 | 0 | 100 | 100 |
| | Main component | - | Phenol | Catechol | Aniline | Phenol | Phenol | 2,6-dimethoxyphenol | 3-pentadecylphenoltriene |
| | Ratio of main component | mol% | 98.6 | 99.4 | 97.6 | 98.9 | 99.6 | 6.66 | 40.7 |
| | Derived from main component | - | Derived from biomass | Derived from biomass | Derived from biomass | Derived from fossil resources | Derived from fossil resources | Derived from biomass | Derived from biomass |
| | Production method | - | Decarboxylation | Decarboxylation | Decarboxylation | Decarboxylation | Reagent | Reagent | Reagent |
| Evaluation results | Contribution to carbon neutrality | - | A | A | A | B | B | A | A |
| | Quality stability | - | A | A | A | A | A | B | B |

As shown in Table 9, the cyclic compounds of each example produced from starting materials containing cyclic carboxylic acid compounds derived from biomass had a sufficiently high concentration of radioactive carbon ¹⁴C and a sufficiently high proportion of the main components having a single molecular structure. In addition, such cyclic compounds were evaluated as having a large contribution to carbon neutrality and stable quality. Therefore, it is recognized that high added value can be imparted to products by using such cyclic compounds as starting materials.

On the other hand, the cyclic compounds of Comparative Examples 1C and 2C are those produced using starting materials derived from fossil resources or reagents derived from fossil resources. Therefore, the cyclic compounds of Comparative Examples 1C and 2C had a low concentration of radioactive carbon ¹⁴C, and the contribution to carbon neutrality was small.

In addition, although the cyclic compounds of Comparative Examples 3C and 4C were biomass-derived reagents, the cyclic compounds were mixtures of various compounds, and thus the proportion of the main components was low. Therefore, the cyclic compounds of Comparative Examples 3C and 4C cannot be said to have high quality stability.

### Industrial Applicability

According to the present invention, a high-quality cyclic compound can be produced from a cyclic carboxylic acid compound in a high yield. In addition, according to another embodiment of the present invention, a cyclic compound that greatly contributes to carbon neutrality and has stable quality can be obtained. Furthermore, according to another embodiment of the present invention, the above cyclic compounds can be produced. Therefore, the present invention has industrial applicability.

### Reference Signs List

S01 Starting material preparation step
S02 Heating step

## Claims

1. A method for producing a cyclic compound, comprising:
a step of causing a decarboxylation reaction in a cyclic carboxylic acid compound by subjecting a starting material containing the cyclic carboxylic acid compound to a heat treatment of heating under a catalyst to obtain a cyclic compound,
wherein the catalyst includes zeolite formed of an aluminum/silicon-containing composite oxide, and
wherein the zeolite has an SiO₂/Al₂O₃ ratio of 7 to 500.

2. The method for producing a cyclic compound according to Claim 1,
wherein an acid amount of the zeolite determined by an NH₃-TPD method is 0.1 to 2.5 [mmol/g].

3. The method for producing a cyclic compound according to Claim 1 or 2,
wherein the catalyst includes at least one of FER type zeolite, MFI type zeolite, BEA type zeolite, and MOR type zeolite.

4. The method for producing a cyclic compound according to Claim 1 or 2,
wherein the zeolite has a pore diameter of 0.4 nm or more and 1.5 nm or less.

5. The method for producing a cyclic compound according to Claim 1 or 2,
wherein the zeolite contains hydrogen.

6. A method for producing a cyclic compound, comprising:
a step of causing a decarboxylation reaction in a cyclic carboxylic acid compound by subjecting a starting material containing the cyclic carboxylic acid compound to a heat treatment of heating in a presence of a non-solid acid to obtain a cyclic compound,
wherein the non-solid acid has an acid dissociation constant pKa of 5.00 or less.

7. The method for producing a cyclic compound according to Claim 6,
wherein the non-solid acid includes an inorganic acid.

8. The method for producing a cyclic compound according to Claim 6 or 7,
wherein the non-solid acid includes an organic acid.

9. The method for producing a cyclic compound according to Claim 8,
wherein the organic acid is an aliphatic carboxylic acid or a derivative of the aliphatic carboxylic acid.

10. The method for producing a cyclic compound according to Claim 6,
wherein the heat treatment is further carried out in a presence of a solid acid.

11. The method for producing a cyclic compound according to Claim 6 or 7,
wherein a temperature of the heat treatment is 150°C to 350°C.

12. The method for producing a cyclic compound according to Claim 1 or 6,
wherein the starting material does not include a solvent.

13. The method for producing a cyclic compound according to Claim 1 or 6,
wherein the cyclic carboxylic acid compound is 4-hydroxybenzoic acid, 3,4-dihydroxybenzoic acid, 4-aminobenzoic acid, or shikimic acid.

14. The method for producing a cyclic compound according to Claim 1 or 6,
wherein the starting material is derived from biomass.

15. A cyclic compound produced by causing a decarboxylation reaction in a cyclic carboxylic acid compound derived from biomass by subjecting a starting material containing the cyclic carboxylic acid compound to a heat treatment,
wherein a concentration of radioactive carbon ¹⁴C in all carbon atoms is 80% to 100%, when a concentration of radioactive carbon ¹⁴C in circulating carbon as of 1950 is set to 100%, and
wherein a proportion of a main component having a single molecular structure is 90 to 100 mol%.

16. The cyclic compound according to Claim 15,
wherein the main component is an aromatic compound derived from biomass.

17. The cyclic compound according to Claim 15 or 16,
wherein a temperature of the heat treatment is 150°C to 350°C.

18. The cyclic compound according to Claim 15 or 16,
wherein the starting material does not include a solvent.

19. The cyclic compound according to Claim 15 or 16,
wherein the cyclic carboxylic acid compound is 4-hydroxybenzoic acid, 3,4-dihydroxybenzoic acid, 4-aminobenzoic acid, or shikimic acid.

20. A method for producing a cyclic compound, comprising:
a step of causing a decarboxylation reaction in a cyclic carboxylic acid compound derived from biomass by subjecting a starting material containing the cyclic carboxylic acid compound to a heat treatment to obtain a cyclic compound,
wherein a concentration of radioactive carbon ¹⁴C in all carbon atoms of the cyclic compound is 80% to 100%, when a concentration of radioactive carbon ¹⁴C in circulating carbon as of 1950 is 100%, and
wherein in the cyclic compound, a proportion of a main component having a single molecular structure is 90 to 100 mol%.
